# EUROPEAN PATENT APPLICATION

(11) **EP 1 344 545 A1**
(43) Date of publication of application: **17.09.2003**
(21) Application number: 01972701.5
(22) Date of filing: 04.10.2001
(51) Int. Cl.: A61M 11/00

(54) **LIQUID ATOMIZER**

(30) Priority: 05.10.2000 JP 2000305687; 03.07.2001 JP 2001202074
(71) Applicant: Omron Corporation, Kyoto-shi, Kyoto-fu, 600-8530 (JP)
(72) Inventor: TERADA, Takao OMRON CORP 801, Minamifudoudou-cho, Kyoto-shi, Kyoto 600-8530 (JP); ASAI, Kei OMRON CORP 801, Minamifudoudou-cho, Kyoto-shi, Kyoto 600-8530 (JP); ARAI, Masato OMRON CORP 801, Minamifudoudou-cho, Kyoto-shi, Kyoto 600-8530 (JP); NISHIMURA, J. OMRON CORP 801, Minamifudoudou-cho, Kyoto-shi, Kyoto 600-8530 (JP); TABATA, Makoto OMRON CORP 801, Minamifudoudou-cho, Kyoto-shi, Kyoto 600-8530 (JP); TANAKA, Shinya OMRON CORP 801, Minamifudoudou-cho, Kyoto-shi, Kyoto 600-8530 (JP); ITOH, Shinichi OMRON CORP 801, Minamifudoudou-cho, Kyoto-shi, Kyoto 600-8530 (JP)
(74) Representative: Kilian, Helmut, Dr.
(86) International application number: JP0108777
(87) International publication number: WO02028459

(57) **Abstract**

A secondary reservoir (16) is filled with a liquid medicine by a fixed amount via a liquid transfer path (18) from a primary reservoir (10) for storing the liquid medicine to be nebulized. When a liquid supply button (40) is slid to left, an operating rod (42) pushes a piston (20) and power supply switch turns on. As piston (20) moves to left, the liquid medicine in secondary reservoir (16) is pushed, thereby moving a valve (22) to left and opening a liquid transfer path (24). Then, a nebulizing part is supplied with the fixed amount of liquid medicine from secondary reservoir (16), and thus performs nebulization. Thus, a liquid nebulizing apparatus is attained, in which a constant amount of liquid is transferred to the nebulizing part to stabilize the nebulization, and in which such a function is realized at low costs.

## Description

### Technical Field

The present invention relates to a liquid nebulizing apparatus with a liquid transport mechanism supplying liquid to a nebulizing part by a constant amount stably.

### Background Art

A conventional portable ultrasonic nebulizing apparatus generally includes, for example, a reservoir for storing liquid (liquid medicine), a oscillation source (an ultrasonic horn oscillator, for example) that is supplied with the liquid in the reservoir at its tip, and a mesh member having numerous fine pores and arranged so as to abut on the tip edge of the oscillation source. The apparatus nebulizes the liquid in the reservoir by the oscillation effect of the oscillation source and the mesh member.

Specifically, the liquid in the reservoir enters the gap between the mesh member and the tip edge of the oscillation source, and by the oscillation effect of the mesh member and the tip edge of the oscillation source, the liquid is discharged as fine spray from the fine pores of the mesh member.

In order to perform a stable nebulizing operation, it is required to supply the nebulizing part stably with a constant amount of liquid from the reservoir. Practically, however, the nebulization can not be stabilized due to fluctuations in the liquid amount supplied of the nebulizing part. Such fluctuation may be invited by the tilt of the apparatus under use, vibrations from surrounding environment, the amount of the liquid remaining in the reservoir or the like. Such an unstable nebulization fails to provide a spray by a constant amount, and therefore it is not preferable to a liquid medicine inhale medication, where a high concentration of liquid medicine should be inhaled by a constant amount every time.

### Disclosure of the Invention

The present invention is made in the light of the forgoing problem. The primary object of the present invention is to provide a liquid nebulizing apparatus in which a constant amount of liquid is transferred to a nebulizing part so that a constant amount of liquid is nebulized every time, and in which such a function is realized at low costs.

Another object of the present invention is to suppress excessive power consumption as well as to reduce the driving time of an oscillation means in an unloaded state, in order to extend the life of the oscillation means.

Still another object of the present invention is to reduce the amount of medicine failed to be inhaled by a user and thus wasted.

In order to achieve the objects above, a liquid nebulizing apparatus according to a first aspect of the present invention includes a primary reservoir for storing liquid to be nebulized; a nebulizing part for nebulizing the liquid in the primary reservoir; a secondary reservoir provided between the primary reservoir and the nebulizing part for measuring the liquid in the primary reservoir by an amount to be transferred to the nebulizing part; a primary liquid transfer part for transferring the liquid in the primary reservoir to the secondary reservoir; a secondary liquid transfer part for transferring the measured liquid in the secondary reservoir to the nebulizer part; and an operation part for operating the secondary liquid transfer part.

In this nebulizing apparatus, the liquid in the primary reservoir is not directly transferred to the nebulizing part, but it is transferred to the secondary reservoir arranged between the primary reservoir and the nebulizing part, where the liquid is measured by the amount per one transfer. Then, thus measured liquid is transferred to the nebulizing part. Accordingly, the liquid is supplied to the nebulizing part at a constant amount irrespective of the tilt of the apparatus in use, vibrations from surrounding environment, or the amount of the liquid remaining in the reservoir, and hence, the nebulization is stabilized.

In a liquid nebulizing apparatus according to the present embodiment, since the primary liquid transfer part and the secondary liquid transfer part send liquid in accordance with the operation of the operation part, respectively, a single operation of the operating part will transfer the liquid in the primary reservoir to the secondary reservoir, and the liquid in the secondary reservoir to the nebulizing part. Thus, the liquid is transferred, measured and transferred again smoothly, providing improved convenience.

Further, if the nebulizing part initiates the liquid nebulizing operation simultaneously with the operation of the operation part, then not only the operability of the apparatus is improved, but also the timing between the liquid transfer to the nebulizing part and the nebulizing operation can be adjusted suitably.

In this case, if the nebulizing part initiates the nebulizing operation before being supplied with the liquid medicine, then the nebulizing part starts nebulization immediately after being supplied with the liquid medicine, thus the initiation of the nebulizing operation is rendered quick and the nebulizing operation is stabilized.

Further, if the liquid is supplied to the secondary reservoir from the primary reservoir when the liquid transfer operation to the nebulizing part in accordance with the operation of the operation part is finished, then next liquid transfer to the nebulizing part in accordance with the operation of the operation part will be performed smoothly.

On the one hand, if the secondary reservoir is closed except for when transferring liquid to the nebulizing part, then the liquid in the secondary reservoir will not be exposed to the outer air, maintaining the liquid (liquid medicine) to be inhaled by a person clean.

On the other hand, if the primary reservoir, the nebulizing part, the secondary reservoir, the primary liquid transfer part and the secondary liquid transfer part are integrally formed as a liquid supply unit with the primary reservoir being removably mounted thereto, then the cleaning of the primary reservoir for storing the liquid is facilitated.

A liquid nebulizing apparatus according to another aspect of the present invention includes a reservoir chamber for storing liquid; a measuring chamber to which a prescribed amount of the liquid is transferred from the reservoir chamber via a liquid transfer port; a nebulizing part for nebulizing the liquid supplied from the measuring chamber via a liquid supply port; a piston for opening and closing the liquid transfer port and pushing the liquid in the measuring chamber to supply the liquid to the nebulizing part via the liquid supply port; and a piston drive part for driving the piston at a constant rate.

By providing the piston drive part as above, the piston can be moved at a constant rate and the liquid such as a liquid medicine can be pushed at a substantially constant force. Therefore, the rate of the liquid to be supplied to the nebulizing part can be made substantially constant, and the liquid can be adhered to the nebulizing part as originally intended.

The liquid nebulizing apparatus preferably includes a valve determining one end of the measuring chamber and supported by an elastic member. The valve is moved by the piston pushing the liquid, thereby opening the liquid supply port to supply the nebulizing part with liquid.

The nebulizing part includes a mesh member and an oscillator and provided with the liquid from the measuring chamber. In this case, a prescribed amount of the liquid can be supplied to a prescribed position on the mesh member by including the piston drive part of the present invention.

The piston driving part includes at least one element selected from the group consisting of elasticity means applying elastic force to the piston, a motor and a solenoid. In other words, the piston drive part of the present invention is the one for driving the piston at a substantially constant force such as an elastic force of a spring or the like, or a force obtained by utilizing electromagnetic force such as a motor or a solenoid.

The liquid nebulizing apparatus above may include lock means for fixing the piston at a position so as to be able to open the liquid transfer port and transfer the liquid into the measuring chamber from the reservoir chamber; and a release switch for releasing the fixed state brought by the lock means and for actuating the piston driving means to drive the piston. Thus, the piston can be driven only by turning the release switch on, supplying the liquid to the nebulizing part in a constant amount by a simple operation.

The liquid nebulizing apparatus above may include lock release sensing means for sensing that the fixed state brought by the lock means is released by the release switch; and nebulizing operation initiating means for initiating a nebulizing operation of the liquid after the lock release sensing means senses that the fixed state brought by the lock means is released by the release switch. In this case, the nebulizing part can automatically be actuated after the locked state of the piston is released. For example, the liquid nebulizing operation can be initiated after the locked state of the piston is released and a prescribed period is passed. Thus, the liquid nebulization can be initiated simultaneously with or immediately after the nebulizing part is supplied with the liquid. Note that, the nebulizing operation initiating means are means for initiating an operation enabling the nebulization of the liquid, including a case where the liquid actually starts to be nebulized by the initiation of the nebulizing operation, and the case where the liquid starts to be nebulized after a prescribed period is passed.

Further, the piston driving part includes a first rod, a second rod for pushing the piston, and a connecting member for connecting the first rod and the second rod. The moving direction of the first rod and that of the second rod are set to be different. Thus, the length of the liquid nebulizing apparatus can be reduced in the moving direction of the piston, since the piston driving part and piston needs not be aligned in a line. In special, by arranging the first rod and the piston as well as the second rod in parallel and driving the first rod and the second rod in the opposite direction, the liquid nebulizing apparatus can be rendered compact.

Further, the liquid nebulizing apparatus above includes a housing having the reservoir chamber inside; and a cylinder having the measuring chamber inside and receiving the piston. In this case, preferably, the cylinder and the piston driving part are removably mounted to the housing.

In the liquid nebulizing apparatus above, the piston driving part has a spiral spring as elasticity means for applying elastic force to the piston. Thus, a constant amount of liquid nebulization can easily be realized by lowering the spring constant of the spiral spring. Additionally, the liquid medicine is prevented from scattering in the nebulizing part, and the apparatus can be rendered compact.

A liquid nebulizing apparatus according to another aspect of the present invention includes a reservoir chamber for storing liquid; a measuring chamber to which a prescribed amount of the liquid is transferred from the reservoir chamber via a liquid transfer port; a nebulizing part for nebulizing the liquid supplied from the measuring chamber via a liquid supply port; a valve determining one end of the measuring chamber and supported by an elastic member for opening and closing the liquid supply port; a piston for opening and closing the liquid transfer port and pushing the liquid in the measuring chamber, thereby moving the valve to open the liquid supply port, whereby the nebulizing part is supplied with the liquid via the liquid supply port; a piston driving part for driving the piston; liquid supply sensing means for sensing initiation of liquid supply to the nebulizing part in advance; and nebulizing operation initiating means for initiating a nebulizing operation of the liquid in response to the liquid supply sensing means sensing initiation of liquid supply to the nebulizing part.

As above, by providing the liquid supply sensing means and the nebulizing operation initiating means, the liquid nebulization operation can automatically be initiated before of after the liquid supply to the nebulizing part is initiated.

The liquid supply sensing means includes a position sensor for sensing the position of at least one of the piston and the valve. In this case, the nebulizing operation initiating means initiates the nebulizing operation of the liquid in response to the position sensor sensing that at least one of the piston and the valve reaches a prescribed position.

By providing the position sensor and the nebulization operation initiating means, the liquid nebulization operation can automatically be initiated when at least one of the piston and the valve reaches a prescribed position. In this case, if the position sensor is set to sense the initiation of liquid supply to the nebulizing part, then the liquid nebulizing operation can be initiated immediately after or before the liquid is supplied to the nebulizing part.

The piston driving part includes a motor and a solenoid. In this case, the nebulizing operation initiating means initiates the a nebulizing operation of the liquid after a driving signal of the motor or the solenoid is input.

The nebulizing part includes a mesh member and an oscillator and supplied with the liquid from the measuring chamber, while the nebulizing operation initiating means includes oscillator driving means for driving the oscillator.

In the liquid nebulizing apparatus above, the piston driving part has a spiral spring as elasticity means for applying elastic force to the piston. Thus, the liquid nebulization by a constant amount can easily be realized by lowering the spring constant of the spiral spring. Additionally, the liquid medicine is prevented from scattering in the nebulizing part, and the apparatus can be rendered compact.

A liquid nebulizing apparatus according to another aspect of the present invention includes a reservoir chamber for storing liquid; a measuring chamber to which a prescribed amount of the liquid is transferred from the reservoir chamber via a liquid transfer port; a nebulizing part having a mesh member and an oscillator for nebulizing the liquid supplied from the measuring chamber via a liquid supply port; a piston for opening and closing the liquid transfer port and pushing the liquid in the measuring chamber, thereby supplying the nebulizing part with the liquid via the liquid supply port; a piston driving part for driving the piston; impedance sensing means for sensing an impedance of the oscillator; oscillation stopping means for stopping oscillation of the oscillator in response to the impedance sensing means sensing that impedance is at most a prescribed value.

The impedance of the oscillator is different when the liquid is present on the mesh member and when not present on the mesh member. Therefore, by sensing the impedance of the oscillator by the impedance sensing means, the presence/absence of the liquid on the mesh member can be sensed. Here, by providing the oscillator stopping means above, the oscillation of the oscillator can be stopped in response to the impedance sensing means sensing that impedance is at most a prescribed value. In other words, the oscillation of the oscillator can be stopped when the absence of the liquid to be nebulized on the mesh member is sensed.

In liquid nebulizing apparatus above, the piston driving part has a spiral spring as elasticity means for applying elastic force to the piston. Thus, the liquid nebulizing by a constant amount can easily be realized by lowering the spring constant of the spiral spring. Additionally, the liquid medicine is prevented from scattering in the nebulizing part, and the apparatus can be rendered compact.

A liquid nebulizing apparatus according to another aspect of the present invention includes a reservoir chamber for storing liquid; a measuring chamber to which a prescribed amount of the liquid is transferred from the reservoir chamber via a liquid transfer port; a nebulizing part for nebulizing the liquid supplied from the measuring chamber via a liquid supply port; a piston for opening and closing the liquid transfer port and pushing the liquid in the measuring chamber, thereby supplying the nebulizing part with the liquid via the liquid supply port; a piston driving part for driving the piston; an inhalation port for a user to inhale the liquid nebulized by the nebulizing part; and inhalation sensing means for sensing the user inhaling from the inhalation port.

By providing the inhalation sensing means as above, it will be possible to sense the user inhaling from the inhalation port. Thus, the nebulization of the liquid or the liquid supply to the nebulizing part in response to the user inhaling from the inhalation port may be possible. Additionally, the shift in the timing of spraying the nebulized liquid and the user's inhale can be reduced.

The liquid nebulizing apparatus above may include nebulizing operation initiating means for initiating a nebulizing operation of the liquid in the nebulizing part in response to the inhalation sensing means sensing an inhalation of the user. The liquid nebulizing apparatus above may include liquid supply means for supplying the liquid to the nebulizing part in response to the inhalation sensing means sensing the inhalation of the user.

In liquid nebulizing apparatus above, the piston driving part has a spiral spring as elasticity means for applying elastic force to the piston. Thus, the liquid nebulizing by a constant amount can easily be realized by lowering the spring constant of the spiral spring. Additionally, the liquid medicine is prevented from scattering in the nebulizing part, and the apparatus can be rendered compact.

### Brief Description of the Drawings

Fig. 1 is an outer perspective view of a liquid nebulizing apparatus according to an embodiment;
Fig. 2 is a partially broken perspective view of a liquid supply unit in liquid nebulizing apparatus according to the embodiment;
Fig. 3 is a vertical cross sectional view of a liquid nebulizing apparatus according to the embodiment;
Fig. 4 is a schematic view showing a state where a secondary reservoir is filled with a liquid medicine in a liquid transfer mechanism in a liquid nebulizing apparatus according to the embodiment;
Fig. 5 is a schematic view showing a state where the liquid medicine in the secondary reservoir is pushed by a piston in the liquid transfer mechanism in the liquid nebulizing apparatus according to the embodiment;
Fig. 6 is a schematic view showing a state where the liquid medicine in the secondary reservoir is transferred to a nebulizing part in the liquid transfer mechanism in the liquid nebulizing apparatus according to the embodiment;
Fig. 7 is a schematic view showing a state where the liquid medicine in the secondary reservoir is completely transferred to the nebulizing part in the liquid transfer mechanism in the liquid nebulizing apparatus according to the embodiment;
Fig. 8 is a schematic view showing a state after the liquid medicine in the secondary reservoir is transferred in the liquid transfer mechanism in the liquid nebulizing apparatus according to the embodiment;
Fig. 9 is a schematic view showing a state where a piston is drawn back after the liquid medicine in the secondary reservoir is transferred in the liquid transfer mechanism in the liquid nebulizing apparatus according to the embodiment:
Fig. 10 is a vertical cross sectional view showing a state corresponding to Fig. 4 in the liquid transfer mechanism in the liquid nebulizing apparatus according to the embodiment;
Fig. 11 is a vertical cross sectional view showing a state corresponding to Fig. 5 in the liquid transfer mechanism in the liquid nebulizing apparatus according to the embodiment;
Fig. 12 is a vertical cross sectional view showing a state corresponding to Fig. 6 in the liquid transfer mechanism in the liquid nebulizing apparatus according to the embodiment;
Fig. 13 is a vertical cross sectional view showing a state corresponding to Fig. 7 in the liquid transfer mechanism in the liquid nebulizing apparatus according to the embodiment;
Fig. 14 is a vertical cross sectional view showing a state corresponding to Fig. 9 in the liquid transfer mechanism in the liquid nebulizing apparatus according to the embodiment;
Fig. 15 is a vertical cross sectional view showing a state in the main part of the liquid nebulizing apparatus according to the embodiment where a power supply switch is turned OFF;
Fig. 16 is a vertical cross sectional view showing a state in the main part of the liquid nebulizing apparatus according to the embodiment where the power on switch is turned ON;
Fig. 17 is a partially broken perspective view showing a state where the nebulizing part is supplied with the liquid medicine from the secondary reservoir in the liquid supply unit of the liquid nebulizing apparatus according to the embodiment;
Fig. 18 is a cross sectional view of the liquid nebulizing apparatus according to a second embodiment of the present invention;
Fig. 19 is an exploded perspective view of the liquid nebulizing apparatus shown in Fig. 18;
Fig. 20 is a partially broken perspective view showing an initial state of the liquid nebulizing apparatus shown in Fig. 18;
Fig. 21 is a partially broken perspective view showing an initial state of the liquid nebulizing apparatus shown in Fig. 18;
Fig. 22 is a partially broken perspective view showing a liquid medicine discharge state of the liquid nebulizing apparatus shown in Fig. 18;
Fig. 23 is a partially broken perspective view showing a liquid medicine discharge complete state of the liquid nebulizing apparatus shown in Fig. 18;
Fig. 24 is a perspective view of a liquid nebulizing apparatus according to a third embodiment of the present invention;
Fig. 25 is an exploded perspective view of the liquid nebulizing apparatus shown in Fig. 24;
Fig. 26 is a partially broken perspective view showing an initial state of the liquid nebulizing apparatus shown in Fig. 24;
Fig. 27 is a partially broken perspective view showing a liquid intake state of the liquid nebulizing apparatus shown in Fig. 24;
Fig. 28 is a partially broken perspective view showing a liquid medicine discharge state of the liquid nebulizing apparatus shown in Fig. 24;
Fig. 29 is a partially broken perspective view showing a liquid medicine discharge complete state of the liquid nebulizing apparatus shown in Fig. 24;
Fig. 30 is a block diagram showing a schematic configuration of a liquid nebulizing apparatus according to a fourth embodiment of the present invention;
Figs. 31A-31D are flow charts for describing an example of an operation where an inhalation sensing is not performed by the liquid nebulizing apparatus according to the fourth embodiment of the present invention;
Fig. 32A is a diagram showing changes in the voltage value of a horn oscillator in a nebulizing operation;
Fig. 32B is a diagram showing changes in impedance of the horn oscillator;
Figs. 33A-33D are flow charts showing an example of operation where inhalation sensing is performed by the liquid nebulizing apparatus according to the fourth embodiment of the present invention;
Fig. 34A is a diagram showing changes in the voltage value of the horn oscillator in a nebulizing operation;
Fig. 34B is a diagram showing changes in impedance of the horn oscillator;
Figs. 35A-35D are flow charts showing another example of operation where inhalation sensing is performed by the liquid nebulizing apparatus according to the fourth embodiment of the present invention;
Fig. 36 is a schematic view showing another example of a piston driving part of the present invention;
Fig. 37 is a schematic view showing still another example of the piston driving part according to the present invention;
Fig. 38 is a schematic view showing still another example of the piston driving part according to the present invention;
Fig. 39 is a schematic view showing still another example of the piston driving part according to the present invention;
Fig. 40 is an exploded perspective view of a liquid nebulizing apparatus according to a fifth embodiment of the present invention;
Fig. 41 is a partially broken perspective view of the liquid nebulizing apparatus shown in Fig. 40;
Fig. 42 is a view for describing a configuration supporting a lever spring;
Fig. 43 is a perspective view showing an initial state of a liquid nebulizing apparatus shown in Fig. 40;
Fig. 44 is a perspective view showing a wind up state of the liquid nebulizing apparatus shown in Fig. 40; and
Fig. 45 shows a relationship between the displacement amount of a spring X and a force applied on a spring F.

### Best Modes for Carrying Out Invention

In the following, the present invention will be described in further detail by embodiments.

### (First Embodiment) .

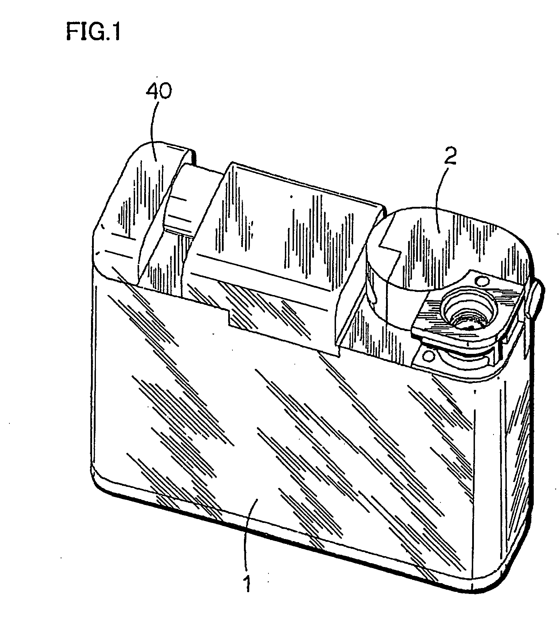

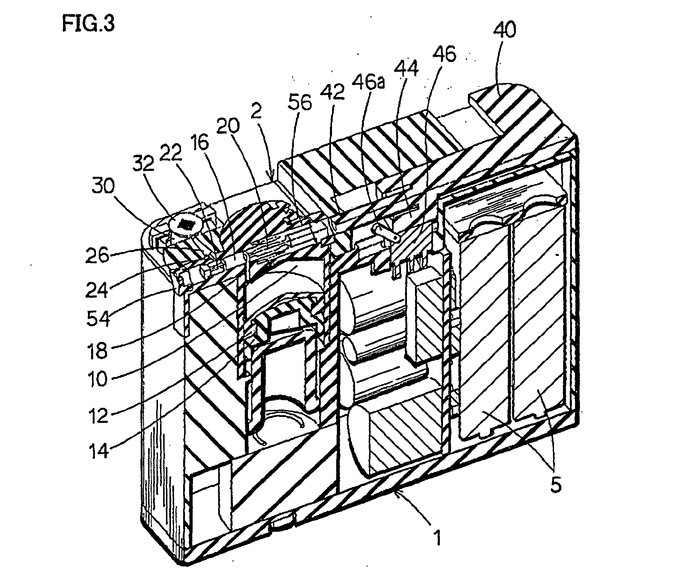

Fig. 1 is an outer perspective view of a liquid nebulizing apparatus according to a first embodiment. The liquid nebulizing apparatus includes a body 1 having a battery accommodation part 5 (see Fig. 3), an electric circuit and the like inside, and a liquid supply unit 2 removably mounted to the body 1.

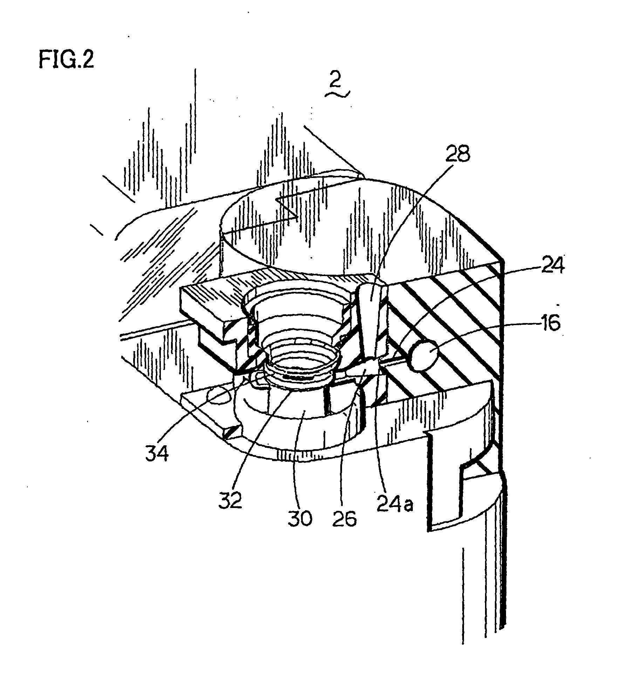

Referring to Fig. 2, which is a partially broken perspective view of liquid supply unit 2, and Fig. 3, which is a vertical cross sectional view showing the interior of the apparatus, a primary reservoir 10 for storing liquid (liquid medicine) to be nebulized is arranged inside the apparatus, and to bottom part of primary reservoir 10, a movable member 12 that is displaceable in upper and lower directions is attached watertight, movable member 12 also being the bottom wall of primary reservoir 10. Movable member 12 is set to be pressurized by a pushing member 14 arranged beneath. Accordingly, the liquid medicine inside primary reservoir 10 is pressurized. A primary liquid transfer part is formed by movable member 12, pushing member 14 and the like. Also, those parts forming primary reservoir 10, movable member 12, and pushing member 14 are removable from liquid supply unit 2, enabling easier cleaning.

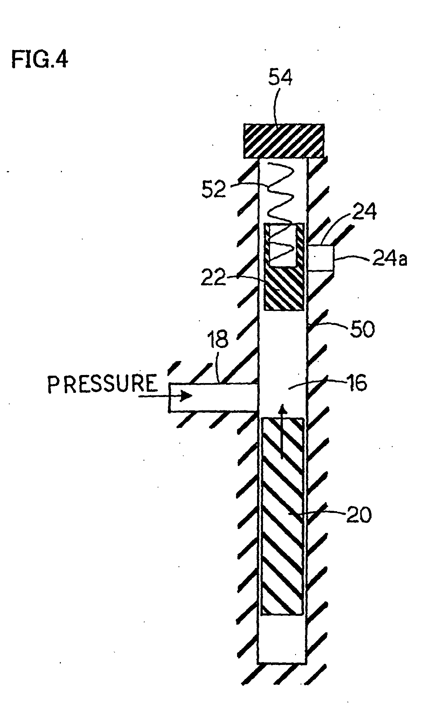

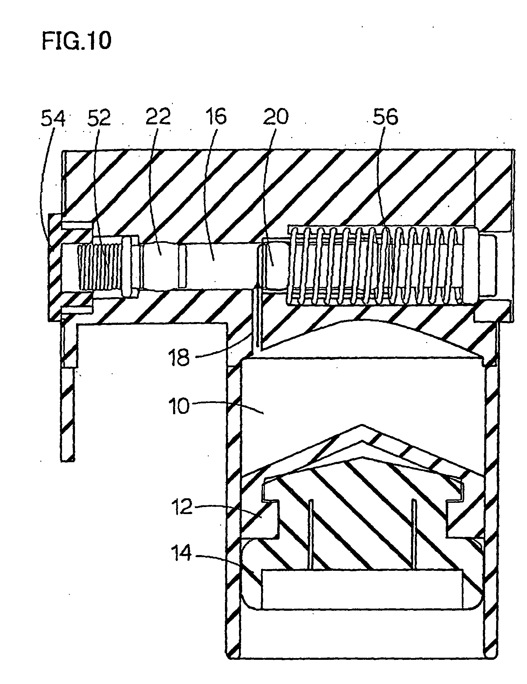

A secondary reservoir 16 measuring the liquid amount to be transferred to a nebulizing part is provided above primary reservoir 10, and this secondary reservoir 16 communicates to primary reservoir 10 via liquid transfer path 18. Secondary reservoir 16 is formed of a cylindrical space extending in horizontal direction (a cylinder), and to one end of this cylindrical space, a piston 20 is movably provided, and a valve 22 is displaceably provided to the other end. A coil spring 56 is inserted into piston 20, which is biased to the right direction in the figure by coil spring 56. Though not shown in Fig. 3, valve 22 is biased to the right direction in the figure by a coil spring 52 (see Figs. 4 and 10), and one end of coil spring 52 engages with a stopper 54 mounted to one end of a cylindrical space. A secondary liquid transfer part is formed by piston 20, valve 22, liquid transfer path 24, coil springs 52, 56 and the like.

Secondary reservoir 16 communicates to liquid transfer path 24, which has an outlet 24a communicating to guide channel 26. Further, a buffer part 28 is provided upper side of guide channel 26 for temporarily storing part of the liquid medicine being transferred. Though guide channel 26 and buffer part 28 are provided on a part different from secondary reservoir 16 or liquid transfer path 24, they may be integrally formed on a common part.

Secondary reservoir 16 communicates to liquid transfer path 24 when transferring liquid, otherwise it is closed. Specifically, even though secondary reservoir 16 communicates to liquid transfer path 18 when the liquid medicine is supplied from primary reservoir 10, the cleanliness of the liquid medicine can be maintained since liquid transfer path 18 is filled with the liquid medicine and the liquid medicine and secondary reservoir 16 will not be exposed to the air.

A horn oscillator 30 is provided close to primary reservoir 10, with its tip facing to guide channel 26. To the tip face of horn oscillator 30, a mesh member 32 having numerous fine pores is abutted, which is pressed against the tip face of horn oscillator 30 by an appropriate force of coil spring 34. The nebulizing part is formed by horn oscillator 30 and mesh member 32.

By this configuration, the liquid medicine in primary reservoir 10 is supplied to the gap between horn oscillator 30 and mesh member 32 via liquid transfer path 18, secondary reservoir 16, liquid transfer path 24, and guide channel 26.

A sliding liquid supply button 40 is provided on body 1 as an operation part, which has a horizontal operating rod 42 for operating piston 20 provided at secondary reservoir 16, and an operating shaft 44 for operating lever 46a of a power supply switch 46 provided below liquid supply button 40. When supplying the liquid medicine to the nebulizing part, liquid supply button 40 is slid to the left side in Fig. 3. Then, operating rod 42 of liquid supply button 40 pushes piston 20, and additionally, operating shaft 44 pushes lever 46a of power supply switch 46, and thus power supply switch 46 turns ON. When liquid supply button 40 is released, piston 20 moves to the right side by the force of coil spring 56 and liquid supply button 40 returns to the state shown in Fig. 3, thereby turning power supply switch 46 OFF.

The timing of turning power supply switch 46 ON is set earlier than the liquid medicine in secondary reservoir 16 reaches the nebulizing part, so that horn oscillator 30 is actuated before the liquid medicine is supplied to the nebulizing part, to initiate the nebulization immediately when the nebulizing part is supplied with the liquid. Thus, the nebulizing operation starts quickly and the nebulizing operation is stabilized.

Since the liquid supply to the nebulizing part is performed by operating the operation part, the nebulizing operation is initiated simultaneously with the operation of the operation part. As used herein, the nebulizing operation includes not only the process of nebulizing the liquid medicine, but also the process of supplying the liquid medicine to the nebulizing part, as will be described in detail later.

Regarding the nebulizing operation of the liquid medicine by the liquid nebulizing apparatus, description will be given referring to schematic views of Figs. 4-9. Those parts identical to the foregoing parts are provided with the same reference characters. Referring to Fig. 4, cylinder 50 (corresponds to the cylindrical space forming secondary reservoir 16 above) communicates to primary reservoir 10 (not shown in Figs. 4-9) via liquid transfer path 18, as well as to guide channel 26 via liquid transfer path 24.

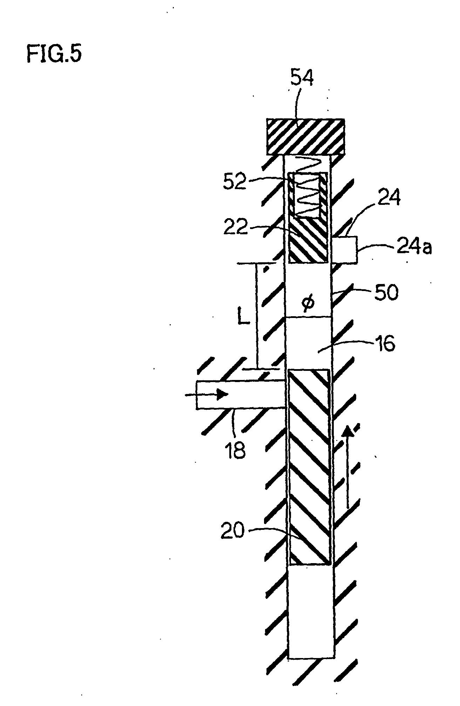

To one end inside cylinder 50, piston 20 also having a valve function for opening/closing liquid transfer path 18 is movably arranged, and to the other end, a valve 22 for opening/closing liquid transfer path 24 is displaceably arranged. Piston 20 is biased downward in the figure by coil spring 56 described above, and valve 22 is similarly biased downward by coil spring 52. One end of coil spring 53 is attached to valve 22, and the other end engages with a stopper 54 attached to the end of cylinder 50. Inside cylinder 50, the space between piston 20 and the valve 22 forms secondary reservoir 16, which is supplied with the liquid medicine from primary reservoir 10. As shown in Fig. 5, the volume of secondary reservoir 16 can be determined by [(*φ*12) 2π · L] where φ is an inner diameter and L is the length of cylinder 50, and set to an appropriate amount required for one transfer in advance.

According to thus structured liquid transfer mechanism, in the state as shown in Fig. 4 where piston 20 opens liquid transfer path 18 while valve 22 closes liquid transfer path 24; since primary reservoir 10 is pressurized with any given pressure, the liquid medicine in primary reservoir 10 flows into secondary reservoir 16 in cylinder 50 via liquid transfer path 18, and thus filling secondary reservoir 16 with a fixed amount of liquid medicine.

When liquid supply button 40 is slid in the state shown in Fig. 4, piston 20 is pushed and moves to the direction shown by an arrow, the upward direction in the figure), and the liquid medicine filled in the secondary reservoir 16 is pressurized by piston 20, which in turn moves valve 22 upward. When piston 20 further moves upward, liquid transfer path 18 is closed by piston 20, and thus shutting the communication between primary reservoir 10 and secondary reservoir 16 (see Fig. 5).

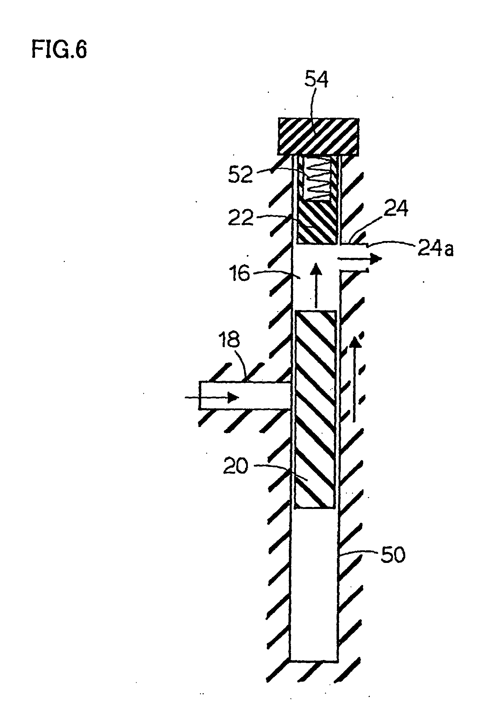

As piston 20 further moves upward, valve 22 moves upward as well, opening liquid transfer path 24. The liquid medicine in secondary reservoir 16 is discharged to guide channel 26 from outlet 24a via liquid transfer path 24, and supplied to nebulizing part (see Fig. 6).

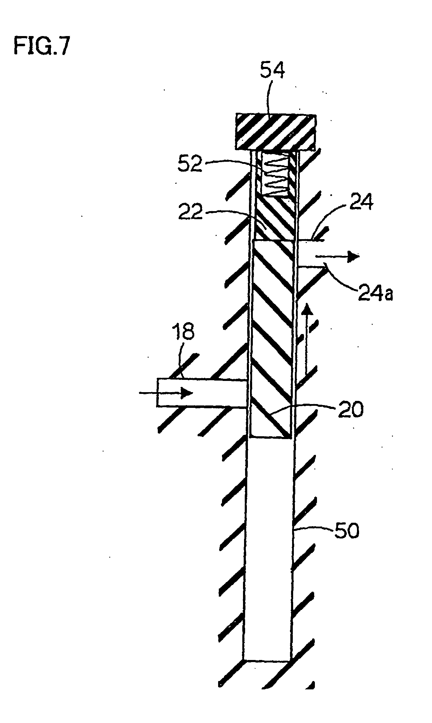

Valve 22 is fixed to any given position (herein to the position where liquid transfer path 24 is completely opened) balanced by the pushing force of piston 20 and discharging pressure of the liquid medicine from outlet 24a. Accordingly, piston 20 moves until contacting to the fixed valve 22, thereafter moves upward together with valve 22, and finally, moves until valve 22 contacts to stopper 54 (see Fig. 7). In this state, the liquid medicine in secondary reservoir 16 is completely discharged from outlet 24a.

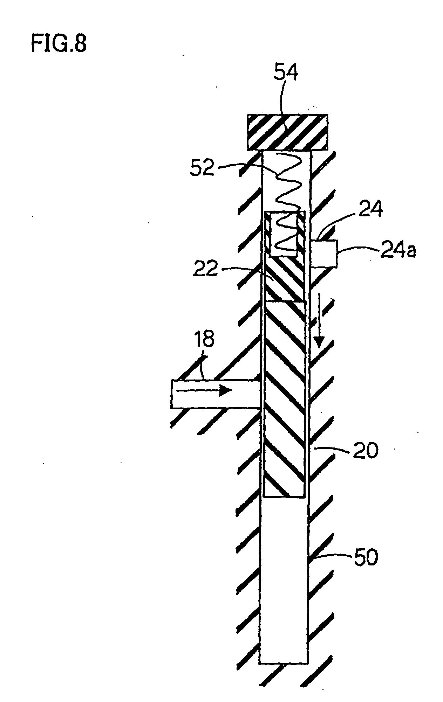

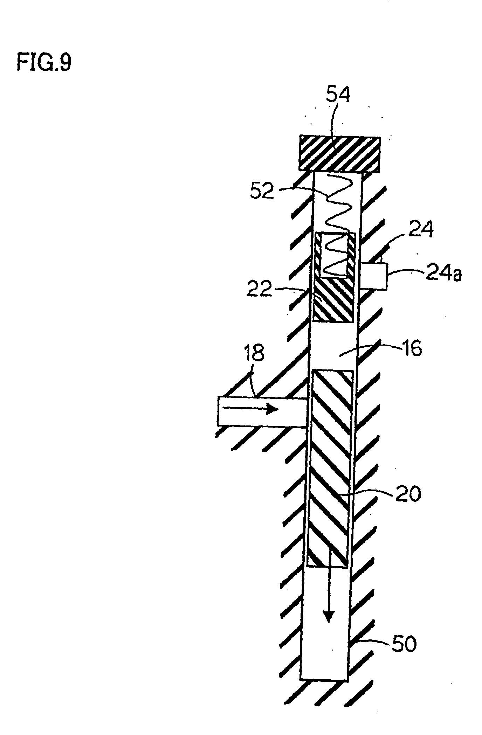

At this time, as liquid supply button 40 is released, piston 20 is pulled back by coil spring 56, and then liquid supply button 40 returns to the original position, with valve 22 returning to the original position as well by the bias force of coil spring 52. As shown in Fig. 8, at first piston 20 and valve 22 moves together downward until valve 22 reaches the position for closing liquid transfer path 24, and thereafter only piston 20 moves downward as shown in Fig. 9.

In this state, as piston 20 further moves downward, secondary reservoir 16 attains a negative pressure state. When piston 20 moves enough to open liquid transfer path 18, the negative pressure of secondary reservoir 16 and the pressure given to the liquid medicine in primary reservoir 10 allows secondary reservoir 16 to be filled with the liquid in primary reservoir 10 (see Fig. 4), whereby a standby state is attained until next liquid supply operation. When liquid supply button 40 is slid again, the operation above is repeated to supply the nebulizing part with the liquid medicine by a fixed amount.

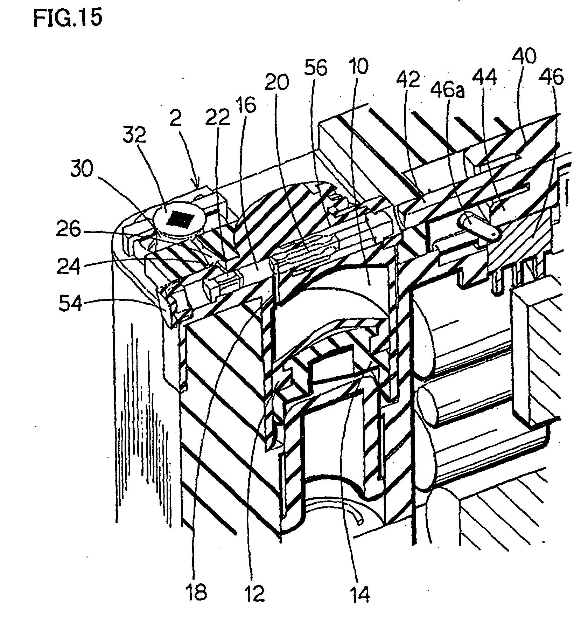

The liquid transfer mechanism as described above is actually arranged in horizontal direction in the apparatus shown in Fig. 3. Next, specific operation thereof will be described referring to Figs. 10-14. First, in the Fig. 10 corresponding to the state shown in Fig. 4, the pressure applied to primary reservoir 10 enables to fill secondary reservoir 16 with the liquid medicine by a fixed amount via liquid transfer path 18. At this time, lever 46a of power supply switch 46 is not operated by operating shaft 44 of liquid supply button 40, and power supply switch 46 is in OFF state (see Fig. 15).

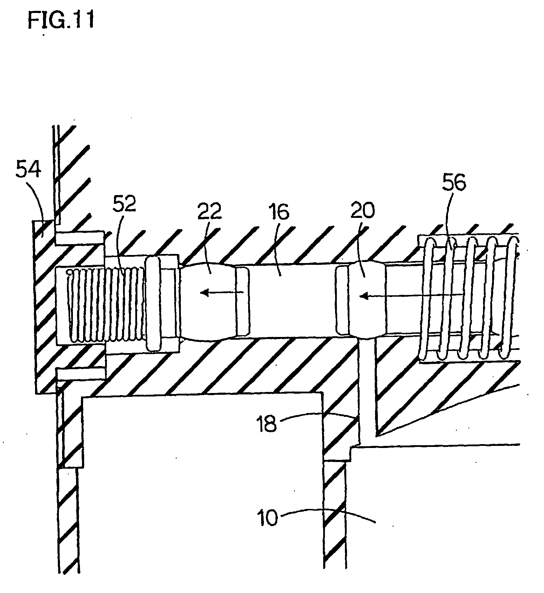

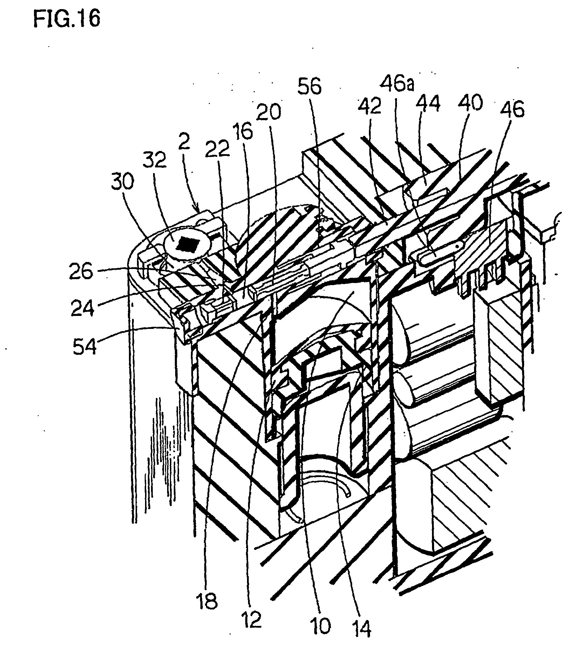

In Fig. 11 corresponding to the state shown in Fig. 5, piston 20 is pushed by operating rod 42 when liquid supply button 40 is slid and thus moves in the direction indicated by an arrow, and valve 22 also moves in the direction indicated by an arrow to close liquid transfer path 18 by piston 20. At this time, as shown in Fig. 16, since liquid supply button 40 moves to left, the lever 46a of power supply switch 46 is pushed by operating shaft 44 to turn power supply switch 46 on, and hence, horn oscillator 30 is actuated before the liquid medicine is supplied to the nebulizing part.

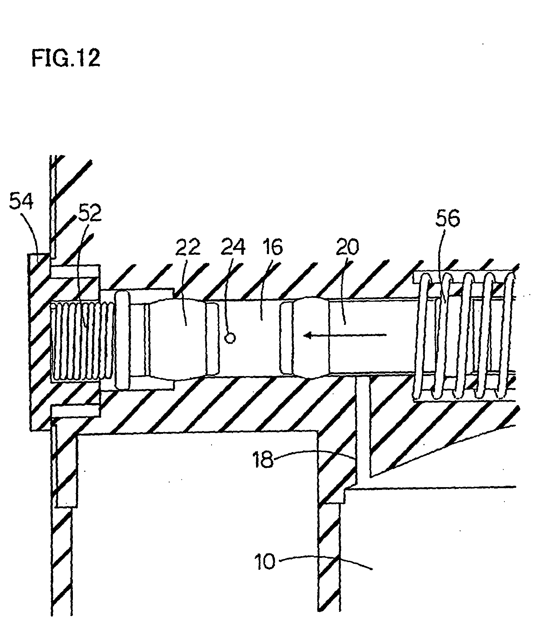

In Fig. 12 corresponding to the state shown in Fig. 6, liquid transfer path 24 is opened by the movement of valve 22, allowing the liquid medicine in secondary reservoir 16 to flow into liquid transfer path 24, and further to be supplied to nebulizing part via guide channel 26.

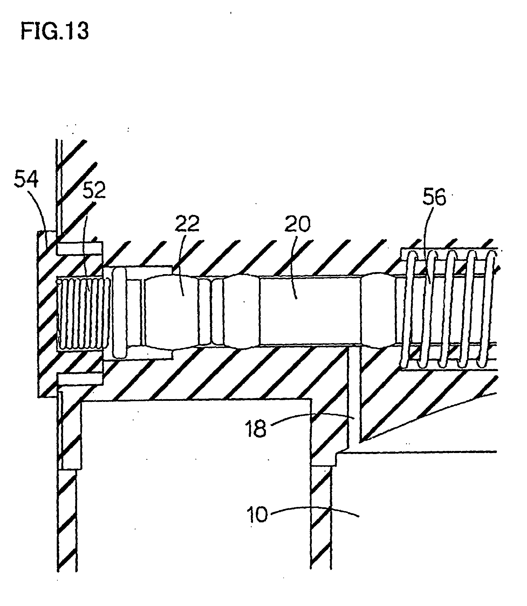

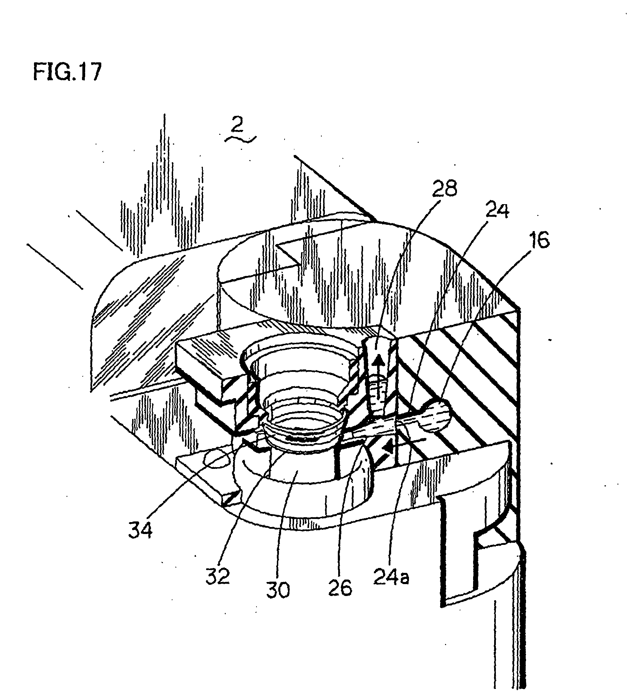

In Fig. 13 corresponding to the state shown in Fig. 7, piston 20 is moved until contacting to valve 22, which allows the fixed amount of the liquid medicine in secondary reservoir 16 to be completely transferred from liquid transfer path 24 to nebulizing part. It should be noted, the liquid medicine is not supplied to the nebulizing part at once, but as shown in Fig. 17, a part of the liquid medicine is temporarily stored in buffer part 28 provided to guide channel 26 so that an appropriate amount of the liquid medicine is supplied to the nebulizing part gradually.

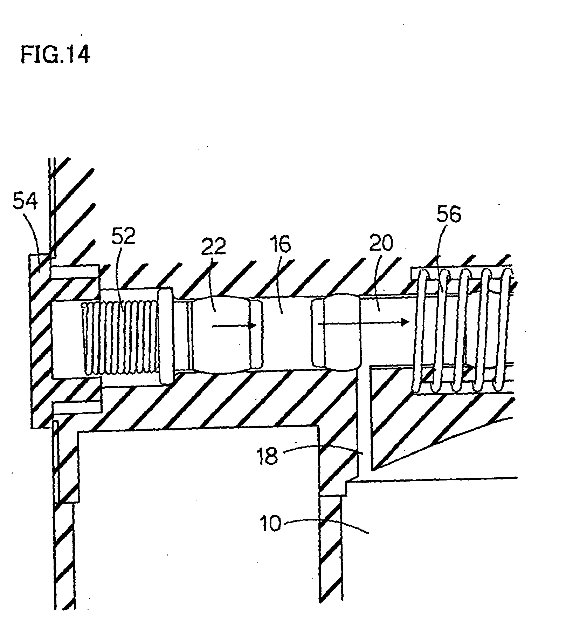

In Fig. 14 corresponding to the state shown in Fig. 9, piston 20 recovers by coil spring 56 as well as valve 22 recovers its original position by coil spring 52, and liquid transfer path 24 is closed by valve 22. Secondary reservoir 16 enters a negative state by the recovery of piston 20, and when piston 20 opens liquid transfer path 18, it returns to the state shown in Fig. 10 to fill secondary reservoir 16 with the liquid medicine from primary reservoir 10, preparing for the next liquid transfer operation.

As has been described, according to the present invention, since the liquid in primary reservoir 10 is not transferred directly to the nebulizing part, but it is temporarily transferred to secondary reservoir 16,provided between primary reservoir 10 and the nebulizing part, where the liquid medicine is measured by the amount per one transfer to the nebulizing part, and thus measured nebulizing liquid is transferred to the nebulizing part, a constant amount of liquid to is supplied to the nebulizing part irrespective of the tilt of the apparatus in use, vibrations from surrounding environment, the amount of the liquid remaining in the reservoir or the like, and hence, the nebulizing operation is stabilized. Additionally, it can be realized by an inexpensive structure.

### (Second Embodiment)

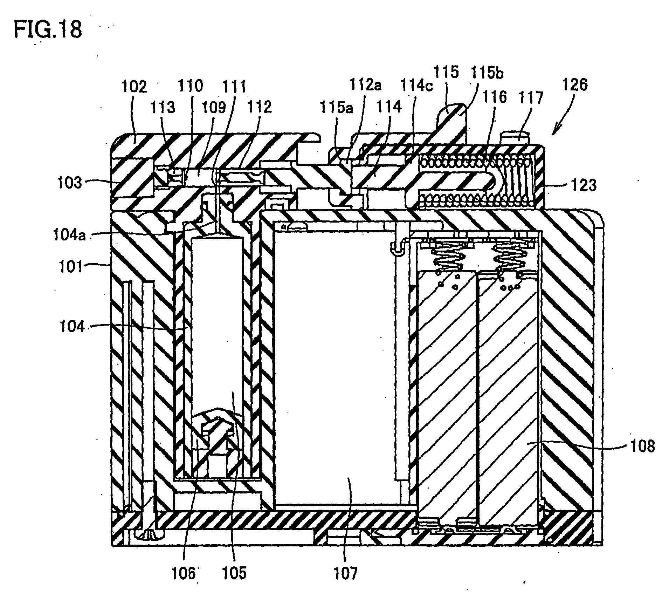

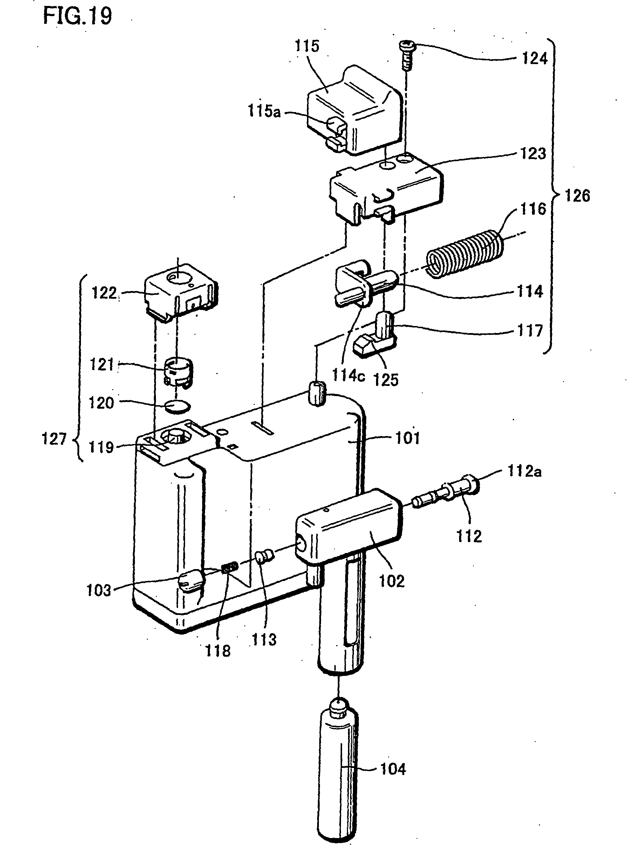

As shown in Figs. 18 and 19, the liquid nebulizing apparatus includes a housing 101, a cylinder 102, a piston driving part 126, and a nebulizing part 127.

As shown in Fig. 18, a battery 108 as a power supply part, and a nebulizing part driving circuit board 107 for driving the nebulizing part are provided inside housing 101. As shown in Fig. 19, a recess is provided to the sidewall of housing 101, to which an ampoule 104 containing a liquid such as a liquid medicine is attached.

As shown in Fig. 18, ampoule 104 has a reservoir chamber 105, a piston 106, and a liquid supply route 104a. A liquid medicine (liquid) is stored in reservoir chamber 105 and supplied to measuring chamber 109, which will be described later, from reservoir chamber 105 via liquid transfer route 104a and liquid transfer port 111.

Cylinder 102 is removably attached to housing 101 to accommodate piston 112. Cylinder 102 has a valve cover 103, measuring chamber 109, a discharge valve 113, a valve spring 118 and a supply port 110.

Piston 112 opens/closes liquid transfer port 111 to supply the liquid medicine from reservoir chamber 105 into measuring chamber 109, and further, supplies the liquid medicine to nebulizing part 127 via liquid supply port 110 by pushing the liquid medicine in measuring chamber 109.

Measuring chamber 109 shown in Fig. 18 is for measuring the liquid medicine by a fixed amount, one wall of measuring chamber 109 being formed by an edge face of piston 112, while the other wall being formed by an edge face of discharge valve 113.

Discharge valve 113 is elastically supported by cylinder 102 via an elastic member such as valve spring 118, and movable to left or right direction (in horizontal direction) in Fig. 18. By pushing the liquid medicine in measuring chamber 109 by piston 112, discharge valve 113 is pressurized, which in turn compresses valve spring 118 and moves discharge valve 113 to the left direction in Fig. 18. Thus, liquid supply port 110 can be opened.

Liquid supply port 110 opens to measuring chamber 109 and communicates to a liquid supply route (not shown) for transferring the liquid medicine from measuring chamber 109 to nebulizing part 127. The liquid medicine supplied to nebulizing part 127 is sprayed after nebulization and thus inhaled by a patient.

Piston driving part 126 drives piston 112 at a constant rate, and being removably mounted to housing 101. This piston driving part 126 has, in the present embodiment, a rod 114, a lever 115, a piston spring 116, a release button 117, a rod case 123, and a screw 124.

Rod 114 extends in the horizontal direction and connected to piston 112, and pushing the piston 112 to the left direction in Fig. 18. Rod 114 has a flange part 114c, to which one end of piston spring 116 is attached or abutted.

Piston spring 116 provides a constant elastic force to piston 112 via rod 114, and thus moves piston 112 in horizontal direction at a constant rate. This piston spring 116 and rod 114 are both accommodated in rod case 123, the other end of piston spring 116 being supported on the wall of rod case 123. Rod case 123 is fixed to housing 101 by screw 124.

Lever 115 is mounted so as to cover one end of rod case 123 and it is used for recovering piston 112 and rod 114 to the original position against the elastic force of piston spring 116.

Lever 115 has a pair of engaging hooks 115a and a projection part (operation part) 115b protruding upward. One end of piston 112 is accommodated between engagement hooks 115a, such that engaging hooks 115a are engaged with a flange part 112a provided at one end of piston.

By pushing protrusion 115b to slide lever 115 to the right side in the Fig. 18, rod 114 and piston 112 moves to the right side in Fig. 18.

Release button 117 is operable by pushing in vertical direction and has a lock hook 125. Lock hook 125 engages with a flange part 114c of rod 114, to limit the movement of rod 114 toward the left direction. Accordingly, when pushing protrusion part 115b by hand to move piston 112 and rod 114 to the right side, piston 112 and rod 114 can be locked at the position where liquid transfer port 111 can be opened to transfer the liquid medicine from reservoir chamber 105 to measuring chamber 109. At this time, piston spring 116 is compressed and elastic force resulted therefrom is applied to rod 114.

For releasing the locked state of piston 112 and rod 114, release button 117 should be pressed downward. Lock hook 125 and flange part 114c of rod 114 are thus disengaged, with piston driving part 126 starting to actuate simultaneously, and piston 112 and rod 114 being moved to left side by the elastic force of piston spring 116 which has been compressed.

As above, by providing lock hook 125 of locking means and release button 117 of a release switch, piston 112 can be driven only by pushing release button 117, thus the fixed amount of liquid medicine can be supplied to nebulizing part 127 by a simple operation.

The liquid nebulizing apparatus as described above preferably includes a lock release sensing part for sensing that the locked state brought by lock hook 125 is released by release button 117, and a nebulizing operation initiation part for initiating the nebulizing operation of the liquid medicine after sensing that the locked state brought by lock hook 125 is released by release button 117.

In this case, nebulizing part 127 can be actuated automatically after releasing the locked state of piston 112, whereby, for example, the nebulizing operation of the liquid medicine can be initiated when a prescribed period is passed after the locked state of piston 112 is released. Thus, nebulizing part 127 can initiate nebulization of the liquid medicine when supplied with the liquid medicine, or immediately after that.

As shown in Fig. 19, nebulizing part 127 includes horn oscillator (ultrasonic oscillator) 119, mesh member 120, mesh member holder 121, and mesh member cap 122.

Mesh member 120 has numerous fine pores, and placed on the tip edge of horn oscillator 119. At this time, mesh member 120 is pressed against the tip edge of horn oscillator 119 by mesh member holder 121 accommodating a coil spring. Mesh member cap 122 is mounted on mesh member holder 121.

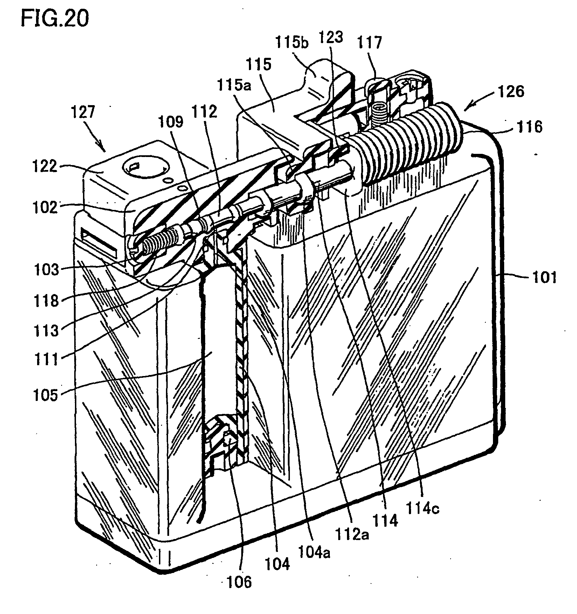

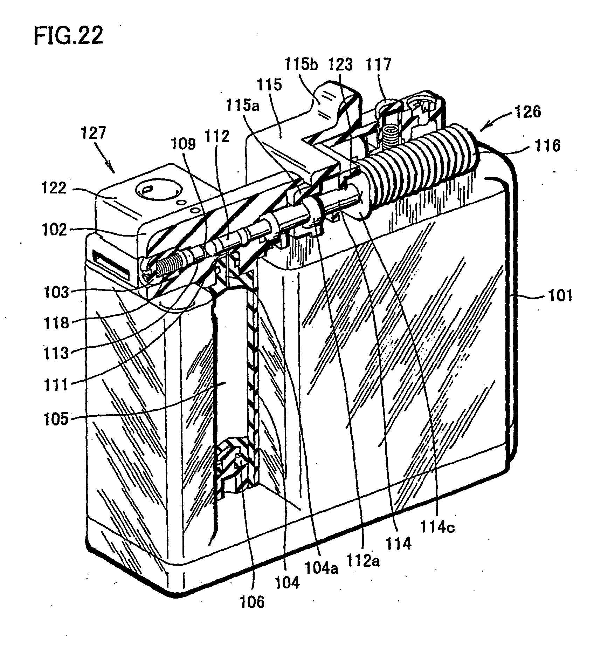

Next, referring to Figs. 20-23, the operation of liquid nebulizing apparatus according to the second embodiment of the present invention will be described.

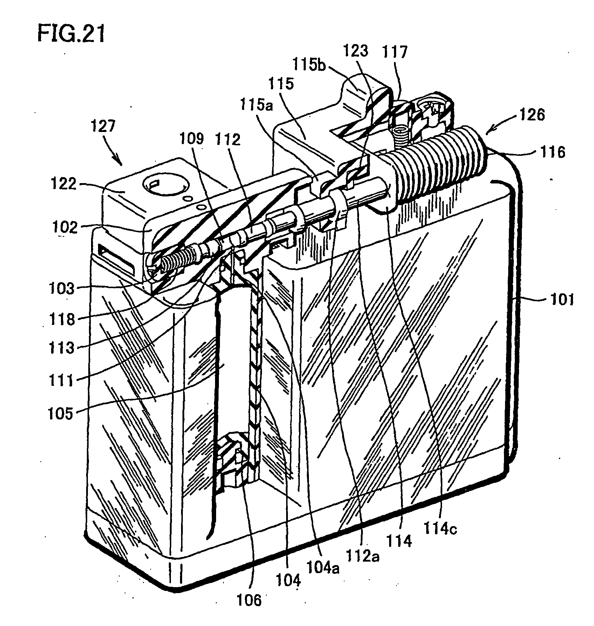

At an initial state shown in Fig. 20, projection part 115b of lever 115 is manually pressed to the right side. Thus, as shown in Fig. 21, lever 115 moves to the right side, with piston 112 and rod 114 together moving to the right side. At this time, piston spring 116 is pressed by flange part 114c of rod 114, and thus compressed.

Additionally, by the movement of the piston 112 toward the right side, liquid transfer route 104a and liquid transfer port 111 communicate to measuring chamber 109, filling measuring chamber 109 under negative pressure with the liquid medicine from reservoir chamber 105.

When lever 115 is moved by a predetermined amount, the extension part extending from one end of flange part 114c of rod 114 to the longitudinal direction of rod 114 engages with lock hook 125 of release button 117, thus locking lever 115, piston 112 and rod 114 at a prescribed position.

Next, release button 117 is pressed downward. Thus, the extension part of flange part 114c and lock hook 125 can be disengaged, and piston 112 and rod 114 can be pressed by the elastic force of piston spring 116 that has been compressed.

As above, by pushing piston 112 and rod 114 by substantially constant force of such an elastic force, as shown in Fig, 22, piston 112 and rod 114 can be moved to the left side at an approximately constant rate. In other words, the liquid medicine in measuring chamber 109 can be pushed by piston 112 with substantially uniform force. Thus, discharge valve 113 is pushed by the liquid medicine, moving to the left side to open liquid supply port 110.

Then, the liquid medicine can be supplied to nebulizing part 127 through liquid supply port 110. Since piston 112 is driven at a substantially constant rate, the rate (momentum) of the liquid medicine supplied to nebulizing part 127 can be kept approximately constant. In other words, the liquid medicine will not be supplied to nebulizing part 127 at an excessive rate and the momentum of the liquid medicine will not be too weak.

As a result, the liquid medicine can be adhered to the position as originally intended in nebulizing part 127 with a desired current, thus the liquid medicine is reliably and efficiently nebulized by nebulizing part 127, reducing the liquid amount remaining in the nebulizing part 127 being failed to be nebulized.

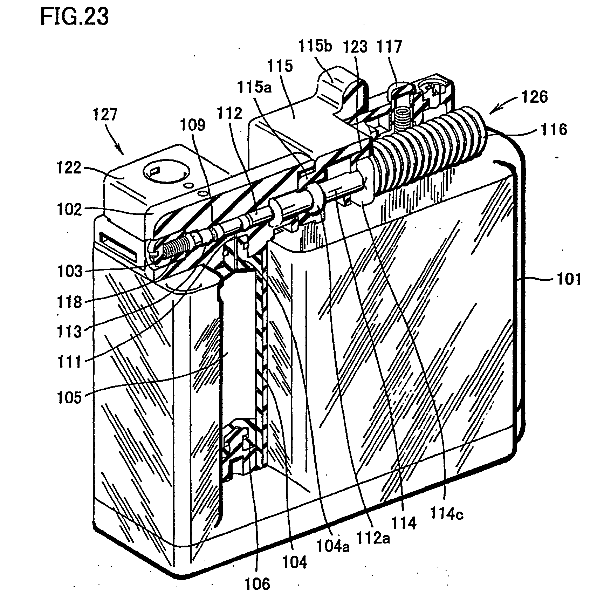

Thereafter, piston 112 moves to the position where it almost abuts on discharge valve 113 as shown in Fig. 23, making the fixed amount of liquid medicine fully discharged from the measuring chamber 109. Accordingly, the discharge of the liquid medicine from measuring chamber 109 is completed.

According to the present embodiment, as compared to the first embodiment, the piston can be driven at a constant rate. This will be described in the following.

In the first embodiment described above, since liquid supply button 40 is operated manually, variations may result in the force applied to liquid supply button 40. The variations in the force applied to liquid supply button 40 may result in variations in moving rate of piston 20, which in turn affect the rate of the liquid medicine supplied into the nebulizing part.

If the rate of the liquid medicine discharged into the nebulizing part is too fast, then the liquid medicine will be scattered in the nebulizing part, while if the rate of the liquid medicine discharged into the nebulizing part is too slow, then the liquid medicine will not reach a predetermined position in the nebulizing part. As a result, the liquid medicine will not be adhered to the nebulizing part against the original intention, increasing the liquid amount remaining in nebulizing part not being nebulized.

A liquid spraying apparatus is disclosed in WO00/66277, which has pushing mechanism to stroke a piston by a prescribed amount accommodated in a liquid bottle by operating a manual operating member, to supply a fixed amount of liquid into a spray chamber by a simple operation. In this case also, since the piston is driven by a manual operation, the liquid medicine will not be adhered to the nebulizing part as the original intention similarly to the aforementioned example, and thus increasing the liquid amount remaining and nebulizing part not being nebulized.

In contrast thereto, according to the present embodiment, since piston 112 and rod 114 are pushed by the elastic force of piston spring 116, piston 112 can be driven at a constant rate. Thus, the problem above can be avoided.

### (Third Embodiment)

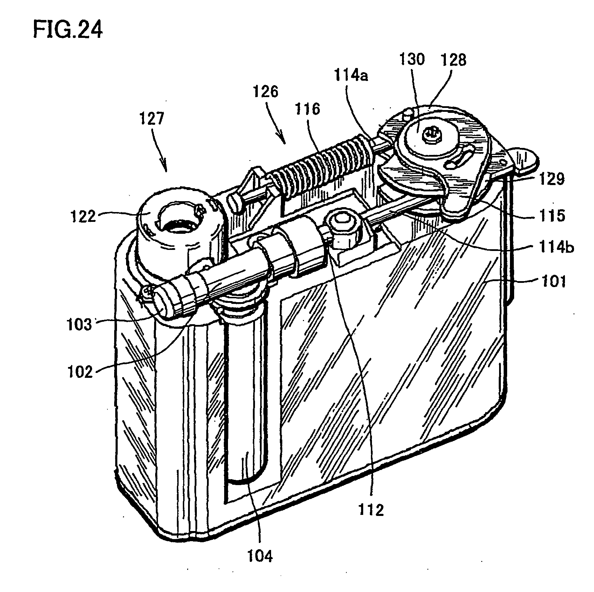

Next, referring to Figs. 24-29, a third embodiment of the present invention will be described.

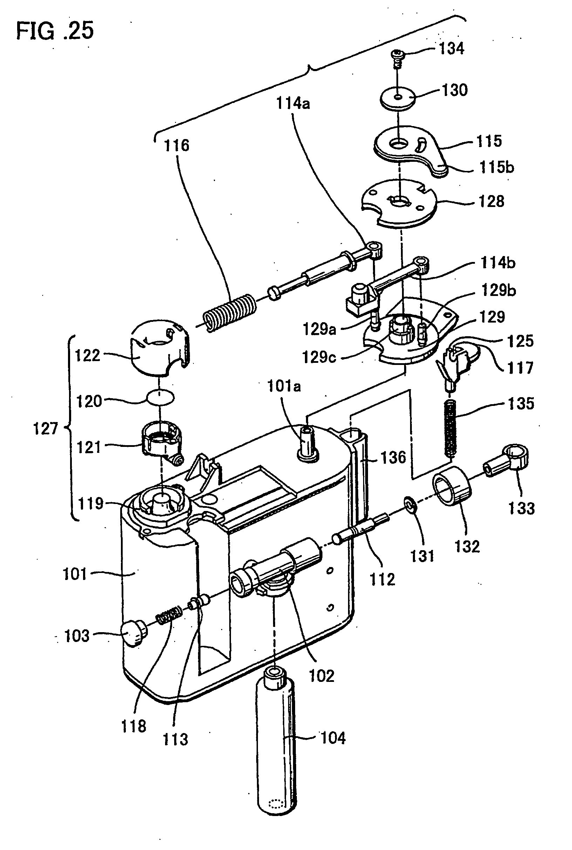

As shown in Figs. 24 and 25, the third embodiment is different from the first embodiment in that the configuration of piston driving part 126 for driving piston 112 is modified. The rest of the configuration is basically the same as that of the second embodiment.

Piston driving part 126 according to the third embodiment includes, as shown in Figs. 24 and 25, rods 114a, 114b, lever 115, piston string 116, release button 117, upper lever plate 128, lower lever plate 129, lever cover 130, and screw 134.

Rod 114a has a small diameter part and a large diameter part at one end, and a ring-form part at the other end. The small diameter part is received by U-shaped recess of a support wall provided on top face of housing 101. Accordingly, the large diameter part and the supporting wall can be engaged, mounting rod 114a to housing 101.

A flange part is provided at the middle portion of rod 114a, and piston spring 116 is provided between the flange part and the supporting wall. This piston spring 116 can push the flange part, driving rod 114a to the right side in Fig. 24.

Rod 114b has a pin part projecting above at one end, and a ring form part at the other end. The pin part is inserted and fixed to the ring-form part of piston coupling rod 113. A tube part is provided at one end of piston coupling rod 113, which is inserted into piston cover 132, and one end of piston 112 is inserted in the tube part via piston stopper 131.

Rods 114a, 114b are connected by upper and lower lever plates 128, 129 that are the connecting members. As shown in Fig. 25, upper and lower lever plates 128, 129 have a perforation hole and a boss part 129c, respectively, for receiving cylindrical part 101a provided on the top face of housing 101, and attached to housing 101 rotatably.

Lower lever plate 129 has a pair of pins 129a, 129b at opposing sides of boss part 129c, and connected to rod 114a by pin 129a and to rod 114b by pin 129b. More specifically, by inserting pins 129a, 129b into the ring-form parts of rod 114a, 114b, respectively, rod 114a, 114b can be connected to lower lever plate 129.

As shown in Figs. 24 and 25, rod 114a and 114b are arranged substantially parallel to each other, with their moving direction being opposite to each other. By arranging the moving directions of rod 114a, 114b opposite to each other, the needs to place piston driving part 126 and piston 112 in a line is eliminated, thus reducing the length of the liquid nebulizing apparatus in the moving direction of piston 112. By arranging rods 114a, 114b substantially parallel to each other, the length of the liquid nebulizing apparatus can be reduced in the width direction also, thus making the liquid nebulizing apparatus compact.

As shown in Fig. 25, lever 115 includes a perforation hole for accommodating cylindrical part 101a at its middle portion, and a long hole for accommodating pin 129b. This lever 115 is in flat shape, and has a protrusion (operation part) 115b protruding to the radial direction. By pushing this protrusion part manually, lever 115 can be turned.

Lever cover 130 is placed on lever 115, and screw 134 is screwed into the cylindrical part 101a from above this lever cover 130. Thus, lever cover 130, lever 115, upper and lower lever plates 128, 129 can be mounted to housing 101.

Release button 117 is elastically supported by release spring 135 accommodated in the spring case 136 provided at a sidewall of housing 101. This release button 117 has lock hook 125, which is inserted into a perforation hole provided to lower lever plate 129. Thus, lower lever plate 129 is locked.

In order to release this locked state, the operation part of release button 117 is manually pushed down. Thus, lock hook 125 moves downward and released from the perforation hole of lower lever plate 129, thereby disengaging the locked states of lower lever plate 129.

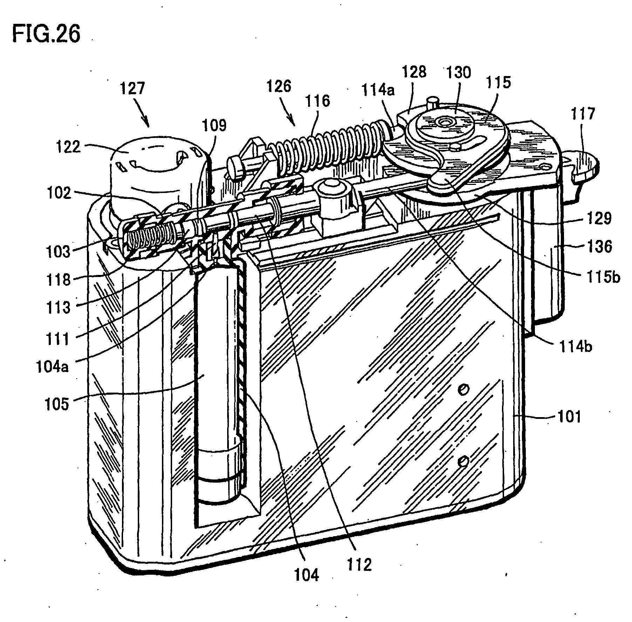

Next, referring to Figs. 26-29, the operation of the liquid nebulizing apparatus according to the present embodiment will be described.

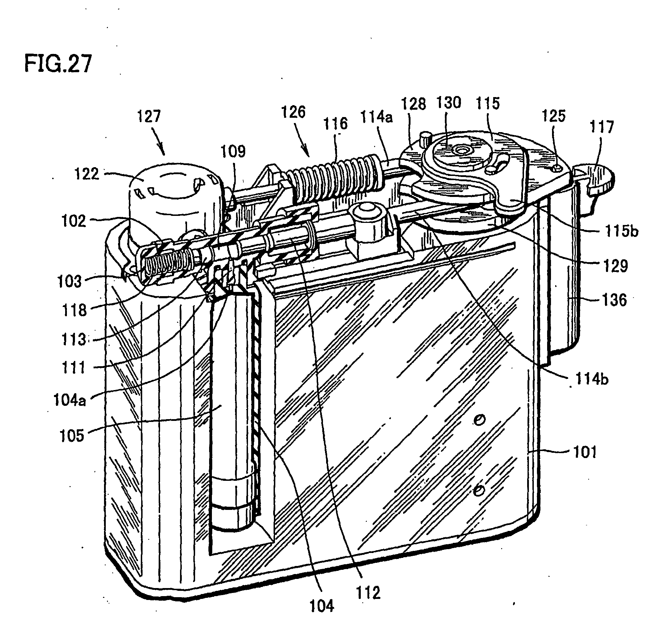

From the initial state shown in Fig. 26, protrusion part 115b of lever 115 is manually turned to left (counterclockwise), thus turning lever 115 by a prescribed angle. Accordingly, upper and lower lever plates 128, 129 as shown in Fig. 27 rotate and rod 114b moves to the right side, and piston 112 moves to the right side as well. At this time, rod 114a moves to the left side, and piston spring 116 is pressed by the flange part of rod 114a and thus compressed.

As piston 112 moves to the right side at least by a prescribed amount, liquid transfer port 111 opens to transfer the liquid medicine from reservoir chamber 105 to measuring chamber 109 as in the second embodiment. At this time, the liquid supply hole is closed by discharge valve 113.

As lever 115 is turned by a prescribed amount, lock hook 125 is inserted into the perforation hole provided at lower lever plate 129 as shown in Fig. 27, thereby locking piston 112 and other parts at a prescribed position.

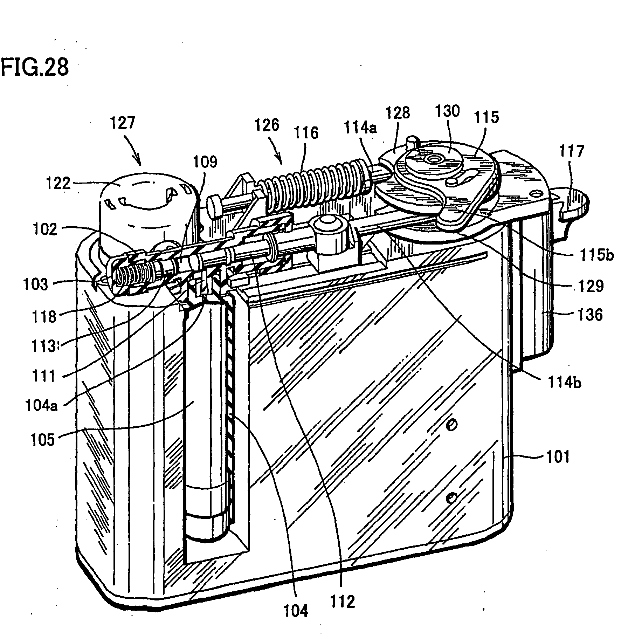

Next, by pushing down release button 117, lock hook 125 is released from the perforation hole provided at lower lever plate 129 as shown in Fig. 28, thereby disengaging lower lever plate 129 and lock hook 125. Thus, rod 114a, rod 114b, and upper and lower lever plates 128, 129 can be driven by the elastic force of piston spring 116 that has been compressed, thereby moving piston 112 to the left side.

As above, by pushing piston 112 with the elastic force of spring, similarly to the second embodiment, the liquid medicine in the measuring chamber 109 can be pushed by piston 112 with substantially uniform force.

Thus, discharge valve 113 is pressurized by the liquid medicine and moves to the left side, thereby opening the supply port 110, through which the liquid medicine is provided to the nebulizing part 127. In the present embodiment also, since piston 112 is driven at an approximately constant rate, the same effect as in the second embodiment can be attained.

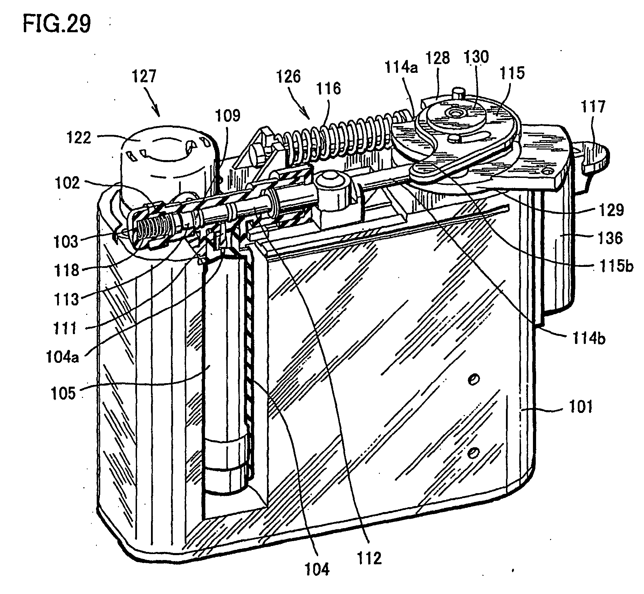

Thereafter, piston 112 moves to the position where it almost abuts on discharge valve 113 as shown in the Fig. 29, supplying nebulizing part 127 with all of the fixed amount of the liquid medicine from measuring chamber 109. Thus, the discharge of the liquid medicine from measuring chamber 109 is completed.

As in the second embodiment, since the present embodiment enables to push piston 112 by the elastic force of piston spring 116, piston 112 can be driven at a constant rate.

### (Fourth Embodiment)

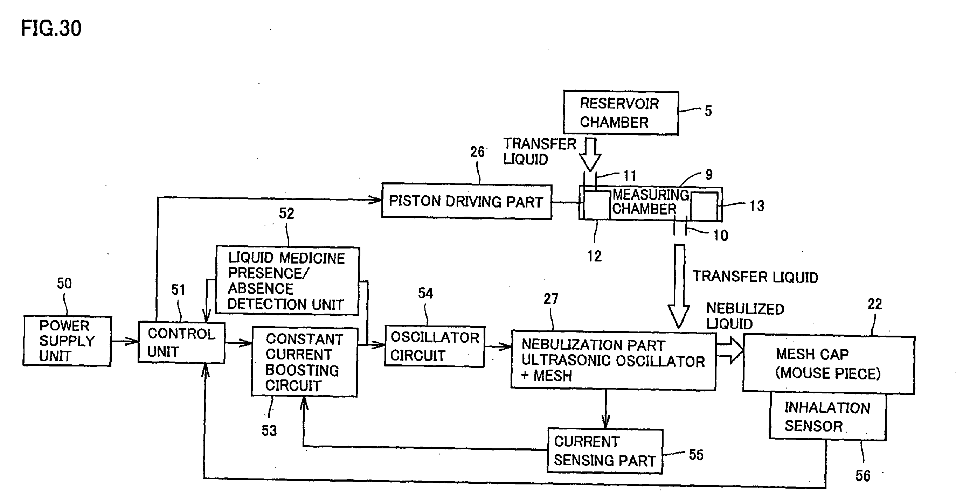

In the following, referring to Figs. 30-39, a fourth embodiment of the present invention and its modification will be described.

Though in the second and third embodiments above, the elastic member such as a spring is applied as driving means of piston 112, piston 112 can be driven with a constant force (rate) using an electromagnetic force such as motor or a solenoid. In the present embodiment, an example using not only a spring but also motor or solenoid is used to drive piston 112 will be described.

As shown in Fig. 30, a liquid nebulizing apparatus according to the present embodiment includes a power supply part 150, a control part 151, a liquid medicine presence/absence sensing part 152, a constant current boosting circuit 153, an oscillator circuit 154, nebulizing part 127, a current sensing part 155, mesh member cap 122, an inhalation sensor 156, piston driving part 126, reservoir chamber 105, liquid transfer port 111, piston 112, measuring chamber 109, discharge valve 113 andiiquid supply port 110.

Power supply part 150 is structured by a dry cell and the like. Control part 151 controls an operation of each part. Constant current boosting circuit 153 boosts the output of power supply part 150, thus constant-current drives oscillator circuit 154 and nebulizing part 127.

As in the second embodiment and others, nebulizing part 127 includes an ultrasonic oscillator (horn oscillator) and a mesh member. Current sensing part 155 senses the current passing through ultrasonic oscillator to attain constant current in constant current boosting circuit 153.

Liquid medicine presence/absence sensing part 152 senses the presence/absence of the liquid medicine in nebulizing part 127 and inputs the sensing result to control part 151, which controls constant current boosting circuit 153 to turn ON/OFF.

The presence/absence of the liquid medicine on the ultrasonic oscillator depends on the impedance of ultrasonic oscillator. Specifically, when the liquid medicine is present on the ultrasonic oscillator, the impedance is high, and when the liquid medicine is not present on the ultrasonic oscillator, the impedance is low.

When the impedance of the ultrasonic oscillator increases, the input impedance of oscillator circuit 154 increases as well, thereby increasing the output voltage of constant current boosting circuit 153. This voltage is input to control part to sense the presence/absence of the liquid medicine on the ultrasonic oscillator.

Inhale sensor 156 senses the inhalation of a patient (user), and in response to the sensing result, the control part 151 controls constant current boosting circuit 53 to turn ON/OFF. This inhalation sensor 156 is provided at or around mesh cap (a mouthpiece: inhalation port) 122, for sensing that the patient is inhaling the liquid medicine nebulized by the nebulizing part 127 from inhalation port. In order to sense the inhalation, a difference in pressure or a change in airflow resulted by the inhalation of the patient should be sensed.

By providing inhalation sensor 156 as above, the inhalation of the patient from the inhalation port can be sensed, and in response thereto, the nebulization of the liquid medicine or the supply of the liquid medicine to nebulizing part 127 can be performed, reducing the shift in the timing of spraying nebulized liquid medicine and the inhalation of the patient.

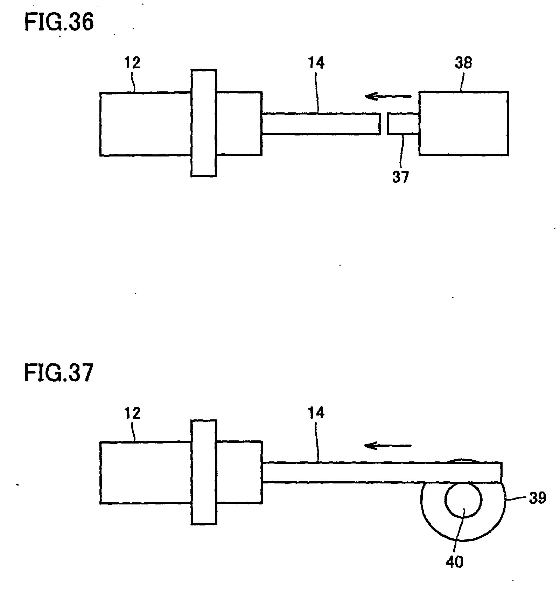

As for piston driving part 126, the configuration as in the second and third embodiments can be employed, and additionally, a configuration shown in Figs. 36-39 can also be employed.

For example, as shown in Fig. 36, piston 112 can be driven using solenoid 138. In this case, by conducting solenoid 138, pin 137 moves to the left side, moving piston 112 to the left side through rod 114. Thus, piston 112 can be driven at a constant rate.

Further, as shown in Fig. 37, piston 112 can be driven using motor 139. In this case, rod 114 is geared, with motor 139 and rod 114 connected with a pinion gear 140. Thus, the driving motor 139 rotates pinion gear 140, thereby moving piston 112 to the left side at a constant rate through rod 114.

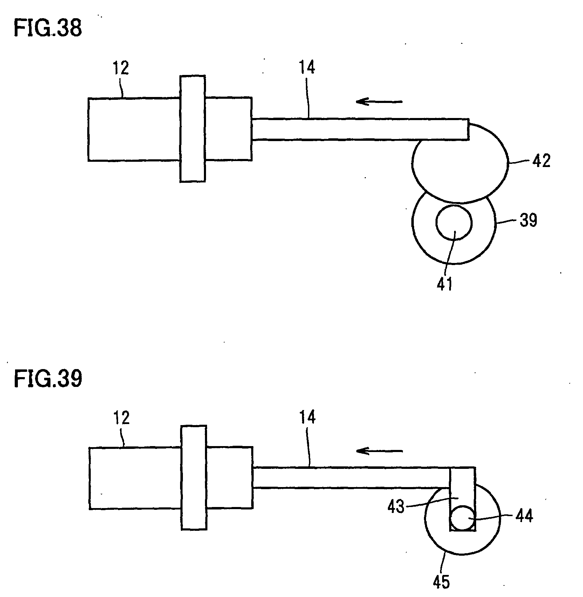

Still further, as shown in Fig. 38, it is also possible to fix rod 114 to gear 142, and to fix gear 141 to the side of motor 139, engaging gear 141 and gear 142. Thus, the power from motor 139 can be transmitted to rod 114 via gears 141 and 142, thereby moving piston 112 to the left side at a constant rate.

Still further, as shown in Fig. 39, piston 112 can be driven using rotary solenoid 145. In this case, the rotation of rotary solenoid 145 rotates arm 143 fixed to shaft 144, whereby rod 114 fixed on arm 143 is pushed to drive piston 112. Thus, piston 112 can be moved to the left side at a constant rate.

Since mesh member cap 122, discharge valve 113, reservoir chamber 105, measuring chamber 109, piston 112, liquid supply port 110, and liquid transfer port 111 may be the same as in the second and third embodiments, their specific description will not be repeated. Additionally, when piston drive part 126 includes a spring, it is not necessary to control piston drive part 126 by control part 151.

Next, the operation of the liquid nebulizing apparatus according to the present embodiment will be described.

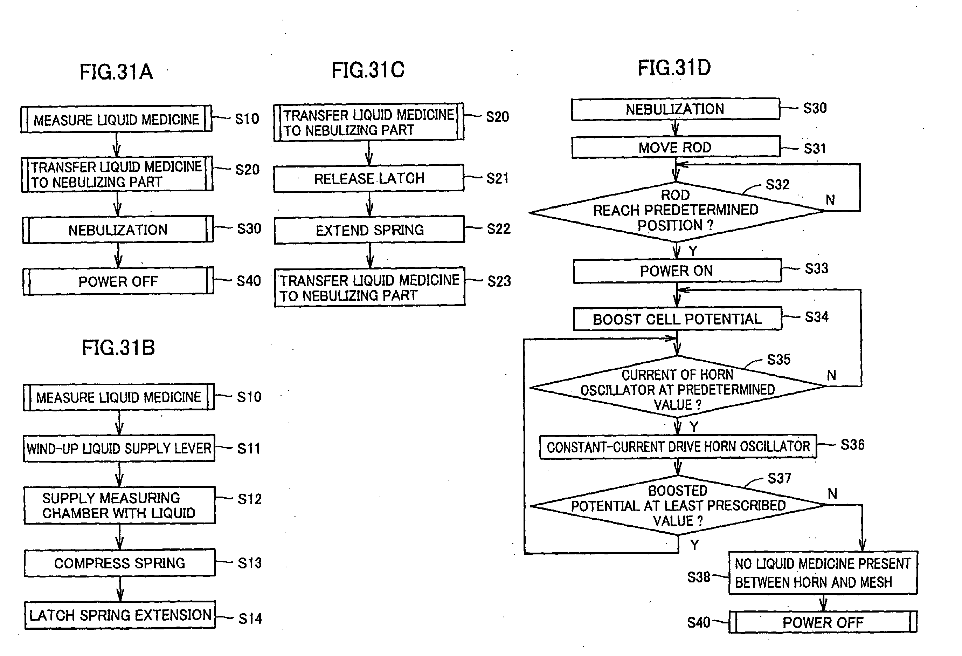

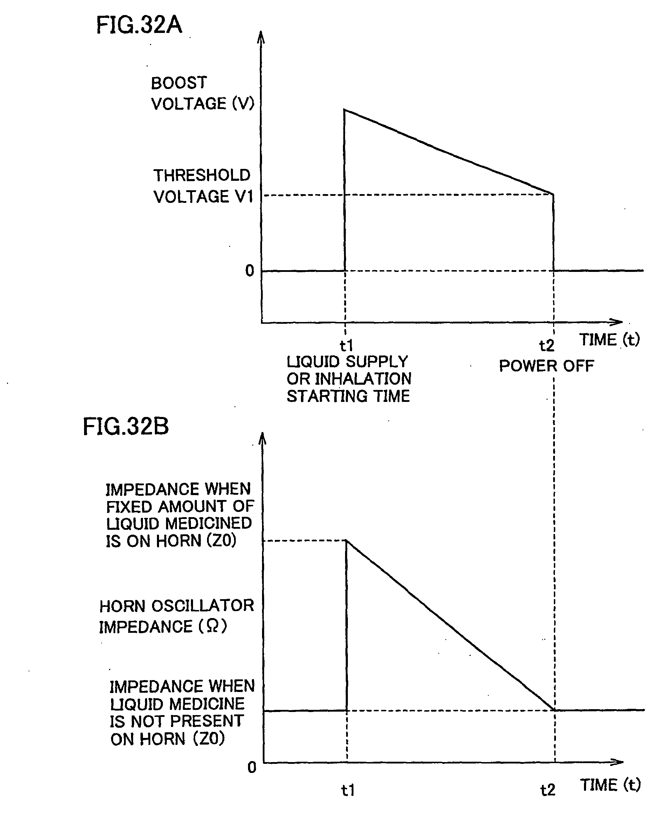

First, referring to Figs. 31A-31D, Figs. 32A and 32B, an operation will be described in which an inhalation sensing is not performed.

As shown in Fig. 31A, at step S10, the liquid medicine is measured by a fixed amount. The manner of measuring the liquid medicine is the same as in the second and third embodiments. Specifically, as shown in Fig. 31B, the liquid supply lever is wound up (step Si 1), and piston 112 is driven to open liquid transfer port 111, thereby supplying measuring chamber 109 with the liquid medicine from reservoir chamber 105 (step S12). At this time, the spring is compressed (step S13), and the extension of the spring is latched in the same manner as in the second and third embodiments (step S 14).

Next, in step S20 in Fig. 31A, the liquid medicine is transferred to nebulizing part 127. The transfer of the liquid medicine is performed by, as shown in Fig. 31C, releasing the latch of the spring by operating the release button as described in the second and third embodiments (step S21), to extend the spring (step S22). Thus, piston 112 is pushed by spring via rod 114, then the liquid medicine in measuring chamber 109 is pushed by piston 112 and discharge valve 113 moves to open liquid supply port 110, thereby transferring the liquid medicine to nebulizing part 127 (step S23).

Next, at step S30 in Fig. 31A, the liquid medicine is nebulized in nebulizing part 127 and sprayed. The spraying of the liquid medicine will be performed in the following manner. First, as shown in Fig. 31D, rod 114 moves by the elastic force of the spring (step S31), the position of rod 114 is sensed (step S32), and in response to sensing the rod 114 reaching a predetermined position, the power supply part 150 is turned ON (step S33).

The position of rod 114 can be detected, by providing a magnet, for example, to an appropriate portion of rod 114 to be sensed by a magnetic sensitive switch (for example a reed relay or a Hall element) provided in the corresponding housing 101. By sensing the position of rod 114 as above, he initiation of liquid supply to the nebulizing part 127 can be sensed in advance.

In response to rod 114 reaching the prescribed position, control part (nebulizing operation initiation part) 151 turns power supply part 150 ON, to start nebulizing operation of the liquid medicine automatically at a desired timing before or after initiating the liquid medicine supply to nebulizing part 127.

Note that, other means can be employed as long as they can sense the initiation of the liquid medicine supply to nebulizing part 127 in advance. For example, it is possible to provide a position sensor for sensing at least one of the positions of piston 112 and discharge valve 113, to function as liquid supply sensing means. The position of discharge valve 113 can be detected, by providing a magnet, for example, to discharge valve 113 to be sensed by a magnetic sensitive switch (for example a reed relay or a Hall element) provided in the corresponding housing 101.

By providing the position sensor as above, the nebulizing operation of the liquid medicine can be initiated automatically, in response to the position sensor sensing one of piston 112 and discharge valve 113 reaching a prescribed position. Thus, the nebulizing operation of the liquid medicine can be initiated immediately after or before the liquid medicine is supplied to nebulizing part 127.

When piston driving part 126 includes a motor or a solenoid, the nebulizing operation initiation means may be the one that initiates the nebulizing operation of the liquid after a driving signal of the motor or the solenoid is input.

After power supply part 150 is turned ON, a cell potential is boosted by constant current boosting circuit 153 (S34). Thereafter, at step S35, the current passing through horn oscillator (ultrasonic oscillator) 119 is sensed to see if it is in a prescribed value, and when the current is at the prescribed value or higher, horn oscillator 119 is driven (step S36) and the nebulizing operation of the liquid medicine is performed.

As shown in Fig. 32B, when the liquid medicine is present on horn oscillator 119, the impedance is relatively high, while it decreases as the liquid medicine is nebulized, and will be the lowest when the liquid medicine is not present on horn oscillator 119.

Accordingly, at step S37 the boosting potential of horn oscillator 119 is sensed to see if it is at a prescribed value or higher. When boosting potential is at most the prescribed value (threshold voltage V1), it is determined that the liquid medicine is not present on horn oscillator 119 (step S38). Then, power supply is turned OFF as indicated in Figs. 31D and 32A (step S40).

In other words, the liquid nebulizing apparatus according to the present invention includes impedance sensing means for sensing the impedance of the oscillator (step S37) and oscillator stopping means for stopping the oscillation of the oscillator in response to the impedance becoming at most the prescribed value (step S40). Thus, the oscillation of horn oscillator 119 can be stopped in response to the absence of the liquid medicine to be nebulized on mesh member 120, suppressing unnecessary power consumption and attaining a long life of horn oscillator 119.

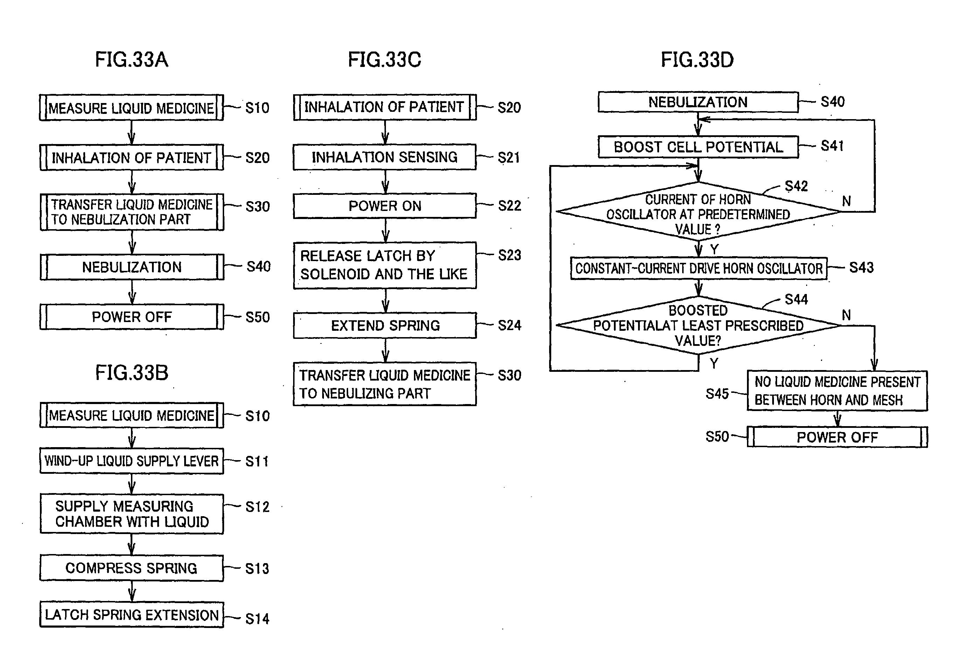

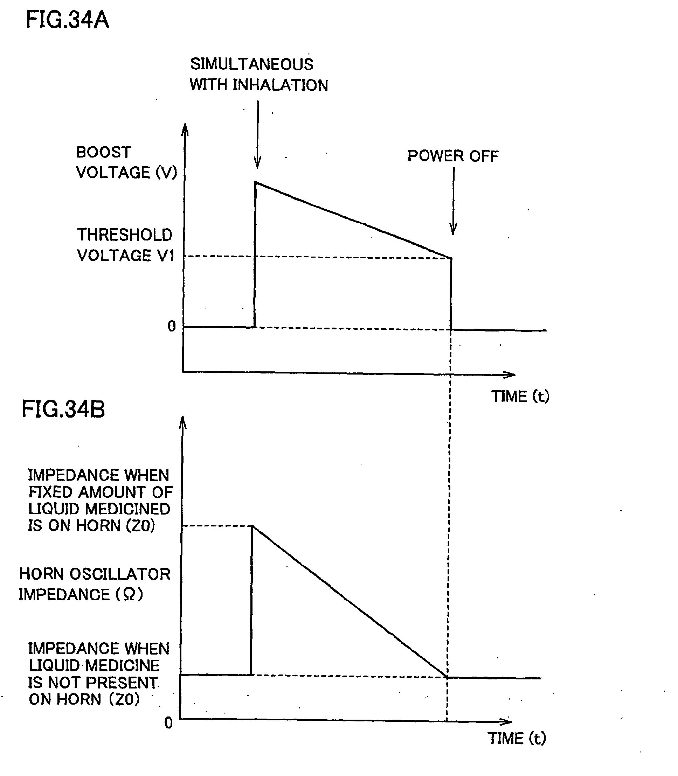

Next, an operation will be described in which an inhalation sensing is performed referring to Figs. 33A-33D, Figs. 34A and 34B.

In an example shown in Fig. 33A, the liquid medicine is measured at step S 10 in a same manner as in the example above, and thereafter the 'inhalation of a patient is sensed at step 520. The inhalation of the patient. is sensed by the inhalation sensor 156 above. As shown in Fig. 33C, in response to sensing the inhalation of the patient (step S21), the power supply is turned ON (step S22), and the spring is released from the latch by the solenoid and the like (step S23) and thus extended (step S24), transferring the liquid medicine to nebulizing part 127 (step S30). In other words, in response to sensing the inhalation of the patient by inhalation sensor 156, the liquid medicine is supplied to nebulizing part 127.

As shown in Fig. 33A, the liquid medicine is nebulized after being transferred to nebulizing part 127, and the liquid medicine thus nebulized is sprayed at step S40. The spraying of the medicine is performed in the same manner as in the example shown in Figs. 31A-31D. Specifically, as shown in Fig. 33D, it is performed through each of the following steps: boosting the cell potential by constant current boosting circuit 153 (step S41); sensing the current passing through horn oscillator 119 (step S42); constant current-driving horn oscillator 119 (step S43); and sensing of boosted potential (step S44).

When boosting potential becomes at most the prescribed value (threshold value V1) as shown in Fig. 34A, it is determined that the liquid medicine to be nebulized is absent on mesh member 120 (step S45), and the power supply is turned OFF (step S50). Thus, in response to the absence of the liquid medicine to be nebulized on mesh member 120, the oscillation of horn oscillator 119 can be stopped.

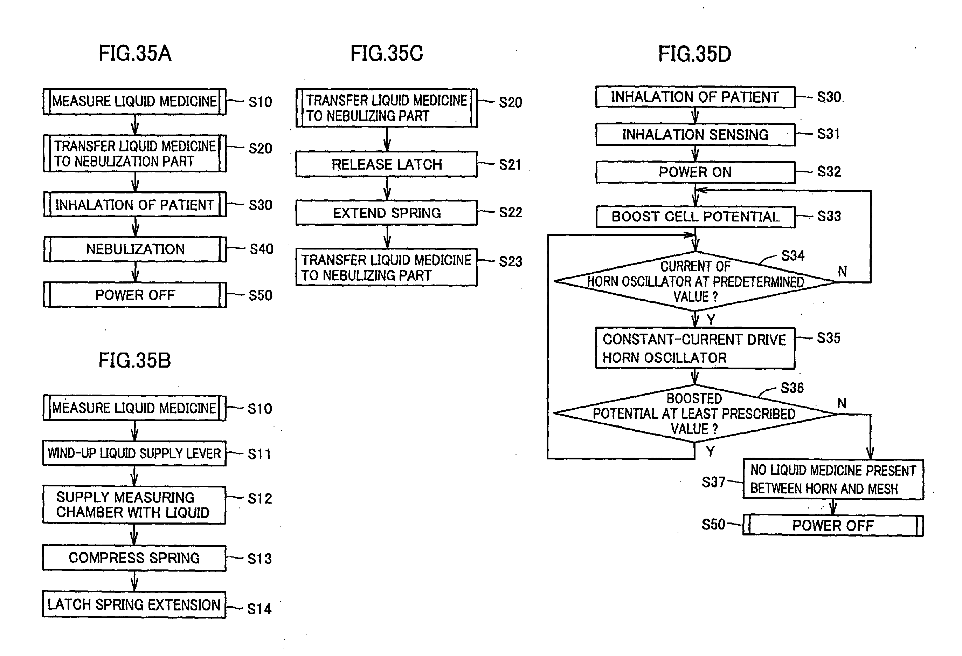

Next, another example of an operation in which the inhalation sensing is performed will be described, referring to Figs. 35A-35D.

As shown in Fig. 35A, the liquid medicine is measured (step S10), and then transferred to nebulizing part 127 (step S20). The manner of measuring liquid medicine is the same as in the example shown in Figs. 33A-33D, and a manner of transferring the liquid medicine to nebulizing part 127 is the same as in the example shown in Figs. 34A and 34B.

Next, the inhalation of the patient is sensed (step S30). As shown in Fig. 35D, the inhalation of the patient is sensed by inhalation sensor 156 (step S31), and in response to the inhalation of the patient, the power supply is turned ON (step S32). In other words, the nebulizing operation of the liquid by nebulizing part 127 is initiated in response to sensing the inhalation of the patient by inhalation sensor 156.

Then, as in the case shown in Figs. 33A-33D, the cell potential is boosted (step S33), the current passing through horn oscillator 119 is sensed (step S34), horn oscillator 119 is constant-current driven (step S35), and the boosting potential is sensed (step S36) and when the boosting potential becomes at most a prescribed value (threshold voltage V1) as shown in Figs. 34A, B, it is determined that the liquid medicine to be nebulized is absent on mesh member 120 (step S37) and the power supply is turned OFF (step S50).

According to the present embodiment, as compared to Japanese Patent National Publication No. 8-502689, the lifetime of the cell or the oscillator can be extended, and also as compared to WO00/66277, the amount of medicine wasted can be reduced. In the following, these features will be described.

The invention described in Japanese Patent National Publication No. 8-502689, is an apparatus for nebulizing liquid by vibrating a thin film, in which the thin film is vibrated for a period longer than the period needed for nebulizing a prescribed amount of the liquid, in order to nebulize of all the prescribed amount of the liquid..

Undesirably, by vibrating the thin film for a long period, not only the lifetime of the cell for vibrating the thin film is shortened, but also not only the lifetime of the oscillator is shortened due to the oscillator being vibrated with no load.

In contrast thereto, since the present embodiment includes liquid supply sensing means 160 and nebulizing operation initiation means 151, the nebulizing operation of the liquid can be automatically initiated at a desired time before or after the liquid supply to nebulizing part 127 is started. Thus, when employing an oscillator for nebulizing the liquid, the driving period in a unloaded state can be reduced, suppressing unnecessary power consumption and extending the life of the oscillator. Additionally, by oscillating the oscillator in advance, just before nebulizing part 127 is supplied with the liquid, even when the liquid supply amount from measuring chamber 109 varies, the load of the oscillator may not be increased excessively and continuous spraying may be attained.

Additionally, since the present embodiment includes the impedance sensing means (step S37) and the oscillator stopping means (step S40), the oscillation of the oscillator can be stopped in response to the absence of the liquid on mesh member 120. Thus, the unnecessary power consumption can be suppressed and the driving of the oscillator in unloaded state can be avoided, extending the life of the oscillator.

Further, in the WO00/66277 above, if the nebulizing part is to be provided with the liquid medicine manually, then the user wishing to inhale the liquid medicine must transfer the liquid medicine to nebulizing part manually, nebulizing and spraying the liquid medicine, and then the user can inhale it. This results in the shift between the timing of spraying the nebulized liquid medicine and the timing of the user's inhalation, and thus the liquid medicine cannot be taken properly. In special, when a small amount of the liquid medicine is to be inhaled, much medicine is wasted by the shift in the timing of spraying and the inhalation.

In contrast thereto, since the present embodiment includes the inhalation sensing means (inhalation sensor 156), the nebulizing operation of the liquid or the liquid supply to the nebulizing part can be performed in accordance with the inhalation of the patient. Thus, the shift in the timing of spraying and the user's inhalation may be smaller, reducing the amount of the medicine not being inhaled by the user and thus wasted.

### (Fifth Embodiment)

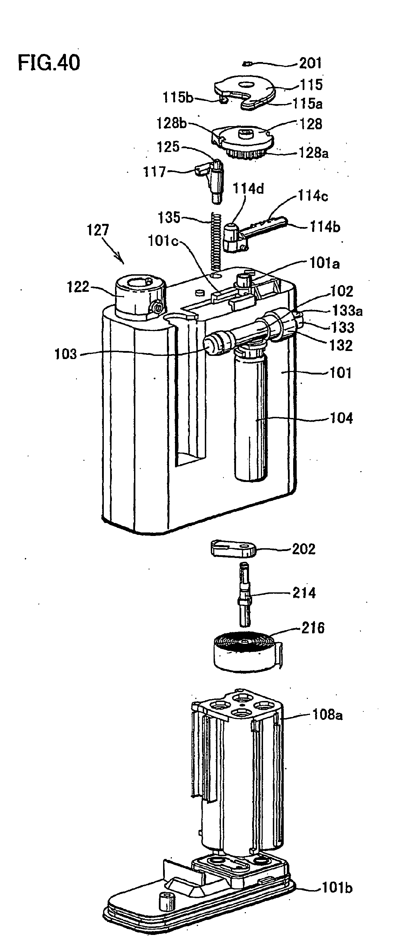

Next, referring to Figs. 40-44, a fifth embodiment of the present invention will be described.

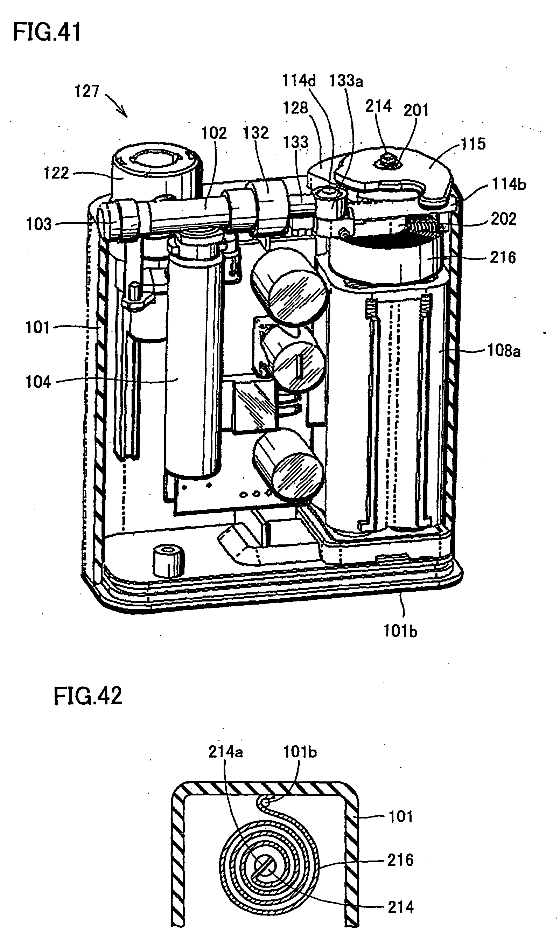

As shown in Figs. 40 and 41, the fifth embodiment is different from the third embodiment in that a spiral spring is used for lever spring 216 in piston driving part 126 for driving a piston (not shown).

Piston driving part 126 includes push rod 114b, wind up lever 115, release lever 117, lever plate 128, release string 135, E-ring 201, switch lever 202, lever shaft 214, and lever spring 216.

Push rod 114b has engaging protruding part 114d and rack 114c, being slidably supported by a guide 101c provided to housing 101. Lever plate 128 has a pinion 128a engaging with rack 114c of push rod 114b, and fitted rotatably to a cylindrical part 101a provided to housing 101. Wind up lever 115 has an operation part 115b that can be manually operated, and engaging protruding part 115c that can be engaged with the recess part 128b of lever plate 128.

Lever shaft 214 is inserted into a perforation hole of cylindrical part 101a from the inside of housing 101, thereby passing through each hole of lever plate 128 and wind up lever 115, and being fitted with E-ring 201 at its tip protruding from wind up lever 115. Additionally, this lever shaft 24 also passes through switch lever 202, whereby switch lever 202 rotates with lever shaft 214. At the lower end of this lever shaft 214, a lever spring 216 formed of spiral spring or the like is provided.

As shown in Fig. 42, lever spring 216 is supported at its inner periphery edge being sandwiched by separating part 214a of lever shaft 214, and at its outer periphery edge being supported by hanging on lib part 101d provided to inside of housing 101.

Release lever 117 has lock hook 125 that can be inserted in a hole provided in lever plate 128, and biased upward by release spring 135.

A battery unit 108a can be inserted into housing 101 from the lower side of lever spring 216, and housing 101 may be closed by a bottom cover unit 101b after inserting battery unit 108a into housing 101.

Piston drive part 126 thus structured can drive piston by the engagement of protrusion part 114d of push rod 114b and the hole of coupling rod 133.

The rest of the configuration is substantially the same as in the third embodiment described above, therefore the identical members are given identical reference characters, and description thereof will not be repeated.

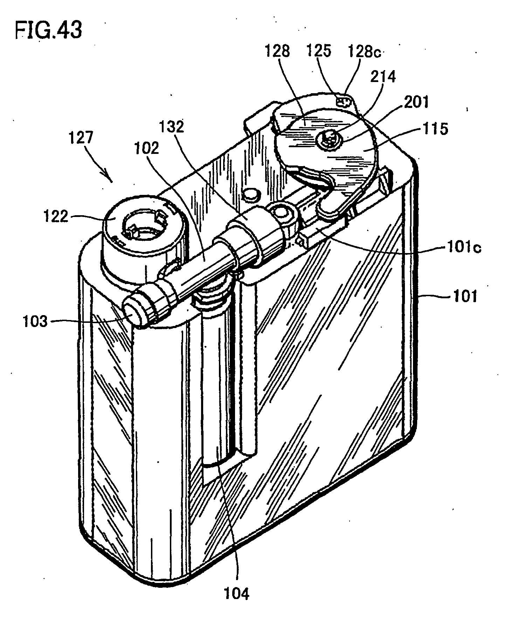

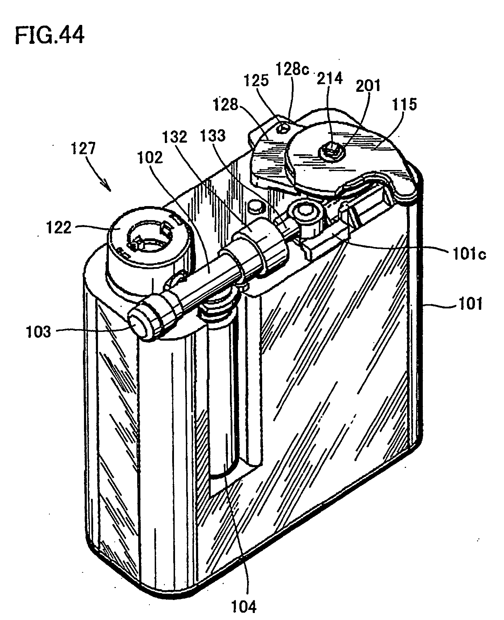

Next, referring to Figs. 43 and 44, the operation of the liquid nebulizing apparatus according to the present embodiment will be described.

From the initial state shown in Fig. 43, the operation part 115b of wind up lever 115 is turned manually to left, (counterclockwise) to turn the wind up lever 115 by a prescribed angle. Thus, lever plate 128 is turned, with push rod 114b and coupling rod 113 sliding to right side, and the piston (not shown) moving to the right side. At this time, the movement of lever shaft 214 winds up lever spring 216 formed of spiral spring, and a biasing force that forces wind up lever 115 to turn to right (clockwise) is generated.

As the piston moves to the right side by a prescribed amount or more, the liquid medicine is transferred from reservoir chamber (not shown) to the measuring chamber (not shown), as in the second and third embodiments. At this time, the liquid supply port (not shown) is closed by the discharge valve (not shown).

As wind up lever 115 is turned by a prescribed amount, the biasing force of release spring 135 inserts lock hook 125 into hole 128c provided to lever plate 128 as shown in Fig. 44, whereby the locked state is attained in which the piston and other parts are locked at a prescribed position.

Next, by pushing down the release lever 117, lock hook 125 disengages from hole 128c provided to lever plate 128, releasing the locked state of lever plate 128 and lock hook 125. Thus, the biasing force of lever spring 216 that has been wound-up turns lever plate 128 to right (clockwise), push rod 114b and coupling rod 133 sliding to the left side.

As above, by pushing the piston (not shown) by the biasing force of lever spring 216, the liquid medicine in the measuring chamber (not shown) can be pressed by the piston (not shown) with an approximately constant force as in the second and third embodiments. Accordingly, as in the second and third embodiments, the nebulizing part 127 can be supplied with liquid medicine.

The manner of the fourth embodiment can be applied to the fifth embodiment as well.

In the present embodiment, as in the second and third embodiments, since the piston can be pushed by the biasing force of lever spring 216, the piston can be driven at a constant rate.

Further, the liquid nebulizing apparatus according to the present embodiment can realize nebulization by constant amount easily, while avoiding scattering of the liquid medicine in nebulizing part 127 and keeping the size of the apparatus smaller, as compared to the second and third embodiments. In the following, these features will be described.

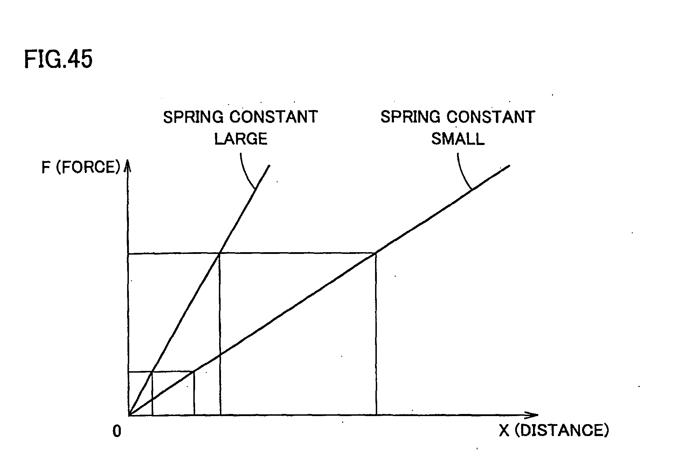

Referring to Fig. 45, normally in a spring, force applied on a spring F and displacement amount X are directionally proportional, and gradient of a straight line indicating the relationship between them will be a spring constant. Here, having a large spring constant, the difference between a force F applied on the spring in wound-up state (the spring being most compressed) and a force F applied on the spring in a state where piston 112 is fully pushed (the spring being most extended) will be large. The difference in the force F is directly proportional to the difference in the rate of the spring pushing the piston 112. Specifically, at the position in wound-up state (the spring being most compressed), the rate of the spring pushing the piston 112 is high, and in a state where piston 112 is fully pushed (the spring being most extended), the rate of the spring pushing piston 112 is lower than that, and thus the amount of changes in the rate becomes large. Accordingly, considering the mass-production of the liquid nebulizing apparatus according to the present embodiment, the variations resulted from individual differences of the springs may hinder to realize the nebulization by a constant amount.

Note that, "the amount of changes in the rate" is sufficiently smaller than in the first embodiment employing manual operation, and thus it can substantially be considered as a constant rate.

The minimum force required as force F applied on the spring in the state where piston 112 is fully pushed must be large enough to sufficiently push discharge valve 113 down against valve spring 118 to push out the liquid medicine from the measuring chamber 109 through liquid supply port 110. Accordingly, when the spring constant is large, larger force F is applied on a spring that is fully wound-up, and the rate the spring pushing the piston 112 will become higher accordingly. Hence, discharge valve 113 is pushed by small amount and the liquid flowing back to ampoule 104 from measuring chamber 109 becomes smaller by that amount. As a result, a phenomenon arises that the liquid amount to be supplied to the nebulizing part is increased. This phenomenon largely fluctuates due to the individual difference of spring, the deterioration of the parts, and variations in the viscosity of the liquid medicine, and thus hinders to realize the nebulization by a constant amount.

Further, with a larger spring constant, the rate of the spring pushing piston 112 will be faster where piston 112 moves past liquid transfer route 104a that communicates to ampoule 104, whereby the liquid medicine is transferred to nebulizing part 127 at high rate, inviting the scattering of the liquid medicine in nebulizing part 127. Hence, it also hinders the nebulization at a constant amount.

If a spring with a smaller spring constant is employed, the difference in the rate of the spring pushing piston 122 becomes smaller and thus the nebulization by a constant amount is facilitated, and the liquid medicine will not be scattered in nebulizing part 127. In this case, however, increasing the force by which a spring pushes piston 112 will increase the displacement amount X of the spring, the length of the overall spring must be increased. Therefore, when a coil spring is employed for piston spring 116 as in the second and third embodiments, since the size is increased in the longitudinal direction of the helical coil spring, it will be difficult to keep the apparatus compact.

In contrast thereto, according to the present embodiment, a spiral spring is employed as lever spring 126. In order to increase the length of the spiral spring, it should be increased in a spiral direction. On the other hand, when increasing in the spiral direction, since the length can be increased in the circumferential direction, the size of spiral spring can be maintained smaller than the coil spring that must be increased in the longitudinal direction. Hence, the liquid nebulizing apparatus according to the present embodiment employing a spiral spring is more suitable for maintaining the apparatus in compact size as compared to the configuration of the second and third embodiments.

As above, by employing a spiral spring having a smaller spring constant as lever spring 216, the liquid nebulizing apparatus according to the present embodiment can easily attain the nebulization by a constant amount, avoiding the scattering of the liquid medicine at nebulizing part 127, in a compact structure.

Although the present invention has been described referring to the embodiments, it is clearly understood that the same is by way of illustration and example only and is not to be taken by way of limitation, the spirit and scope of the present invention being limited only by the terms of the appended claims, including all modifications in the equivalents of the claims and in the scope of the claims.

### Industrial Applicability

The present invention can advantageously be applied to a liquid nebulizing apparatus for stabilizing the liquid amount being transferred to a nebulizing part for stabilizing the nebulization, and for realizing it at low costs.

## Claims

1. A liquid nebulizing apparatus, comprising:
a primary reservoir for storing liquid to be nebulized;
a nebulizing part for nebulizing the liquid in said primary reservoir; a secondary reservoir provided between said primary reservoir and said nebulizing part for measuring the liquid from said primary reservoir by an amount to be transferred to said nebulizing part;
a primary liquid transfer part for transferring the liquid in said primary reservoir to said secondary reservoir;
a secondary liquid transfer part for transferring the measured liquid in said secondary reservoir to said nebulizer part; and
an operation part for operating said secondary liquid transfer part.

2. The liquid nebulizing apparatus according to claim 1, wherein said primary liquid transfer part and said secondary liquid transfer part respectively transfer the liquid relative to an operation of said operation part.

3. The liquid nebulizing apparatus according to claim 1, wherein said nebulizing part initiates a liquid nebulizing operation simultaneously with an operation of said operation part.

4. The liquid nebulizing apparatus according to claim 3, wherein said nebulizing part is set to initiate a nebulizing operation before said nebulizing part is supplied with the liquid in accordance with the operation of said operation part.

5. The liquid nebulizing apparatus according to claim 1, wherein said secondary reservoir is set to be supplied with the liquid from said primary reservoir when the liquid transfer operation to said nebulizing part in accordance with the operation of said operation part is completed.

6. The liquid nebulizing apparatus according to claim 1, wherein said secondary reservoir is closed except for when transferring liquid to said nebulizing part.

7. The liquid nebulizing apparatus according to claim 1, wherein said primary reservoir, said nebulizing part, said secondary reservoir, said primary liquid transfer part and said secondary liquid transfer part are integrally formed as a liquid supply unit, said primary reservoir being removable from said liquid supply unit.

8. A liquid nebulizing apparatus, comprising:
a reservoir chamber for storing liquid;
a measuring chamber to which a prescribed amount of said liquid is transferred from said reservoir chamber via a liquid transfer port;
a nebulizing part for nebulizing said liquid supplied from said measuring chamber via a liquid supply port;
a piston for opening and closing said liquid transfer port and pushing said liquid in said measuring chamber to supply said nebulizing part with said liquid via said liquid supply port; and
a piston drive part for driving said piston at a constant rate.

9. The liquid nebulizing apparatus according to claim 8, further comprising
a valve determining one end of said measuring chamber and supported by an elastic member, said valve being moved by said piston pushing said liquid, thereby opening said liquid supply port to supply said nebulizing part with said liquid.

10. The liquid nebulizing apparatus according to claim 8, wherein said nebulizing part includes a mesh member and an oscillator and supplied with said liquid from said measuring chamber.

11. The liquid nebulizing apparatus according to claim 8, wherein
said piston driving part includes at least one element selected from the group consisting of elasticity means for applying elastic force to said piston, a motor and a solenoid.

12. The liquid nebulizing apparatus according to claim 8, further comprising:
lock means for fixing said piston at a position so as to be able to open said liquid transfer port and transfer said liquid into said measuring chamber from said reservoir chamber; and
a release switch for releasing the fixed state brought by said lock means and for actuating said piston driving part to drive said piston.

13. The liquid nebulizing apparatus according to claim 12, further comprising:
lock release sensing means for sensing that the fixed state brought by said lock means is released by said release switch; and
nebulizing operation initiating means for initiating a nebulizing operation of said liquid after said lock release sensing means senses that the fixed state brought by said lock means is released by said release switch.

14. The liquid nebulizing apparatus according to claim 8, wherein said piston driving part includes a first rod, a second rod for pushing said piston, and a connecting member for connecting said first rod and said second rod, and
a moving direction of said first rod and a moving direction of said second rod are set to be different.

15. The liquid nebulizing apparatus according to claim 8, further comprising:
a housing having said reservoir chamber inside; and
a cylinder having said measuring chamber inside and receiving said piston; wherein
said cylinder and said piston driving part are removably mounted to said housing.

16. The liquid nebulizing apparatus according to claim 8, wherein said piston driving part has a spiral spring as elasticity means for applying elastic force to said piston.

17. A liquid nebulizing apparatus, comprising:
a reservoir chamber for storing liquid;
a measuring chamber to which a prescribed amount of said liquid is transferred from said reservoir chamber via a liquid transfer port;
a nebulizing part for nebulizing said liquid supplied from said measuring chamber via a liquid supply port;
a valve determining one end of said measuring chamber and supported by an elastic member for opening and closing said liquid supply port;
a piston for opening and closing said liquid transfer port and pushing said liquid in said measuring chamber, thereby moving said valve to open said liquid supply port, whereby said nebulizing part is supplied with said liquid via said liquid supply port;
a piston driving part for driving said piston;
liquid supply sensing means for sensing initiation of liquid supply to said nebulizing part in advance; and
nebulizing operation initiating means for initiating a nebulizing operation of said liquid in response to said liquid supply sensing means sensing initiation of liquid supply to said nebulizing part.

18. The liquid nebulizing apparatus according to claim 17, wherein said liquid supply sensing means includes a position sensor, for sensing a position of at least one of said piston and said valve;
said nebulizing operation initiating means initiates the nebulizing operation of said liquid in response to said position sensor sensing that at least one of said piston and said valve reaches a prescribed position.

19. The liquid nebulizing apparatus according to claim 17, wherein said piston driving part includes a motor and a solenoid; and
said nebulizing operation initiating means initiates the nebulizing operation of said liquid after a driving signal of said motor or said solenoid is input.

20. The liquid nebulizing apparatus according to claim 17, wherein said nebulizing part includes a mesh member and an oscillator and supplied with said liquid from said measuring chamber, and
said nebulizing operation initiating means includes oscillator driving means for driving said oscillator.

21. The liquid nebulizing apparatus according to claim 17, wherein said piston driving part has a spiral spring as elasticity means for applying elastic force to said piston.

22. Aliquid nebulizing apparatus, comprising:
a reservoir chamber for storing liquid;
a measuring chamber to which a prescribed amount of said liquid is transferred from said reservoir chamber via a liquid transfer port;
a nebulizing part having a mesh member and an oscillator for nebulizing said liquid supplied from said measuring chamber via a liquid supply port;
a piston for opening and closing said liquid transfer port and pushing said liquid in said measuring chamber, thereby supplying said nebulizing part with said liquid via said liquid supply port;
a piston driving part for driving said piston;
impedance sensing means for sensing an impedance of said oscillator;
oscillation stopping means for stopping oscillation of said oscillator in response to said impedance sensing means sensing that impedance is at most a prescribed value.

23. The liquid nebulizing apparatus according to claim 22, wherein said piston driving part has a spiral spring as elasticity means for applying elastic force to said piston.

24. A liquid nebulizing apparatus, comprising:
a reservoir chamber for storing liquid;
a measuring chamber to which a prescribed amount of said liquid is transferred from said reservoir chamber via a liquid transfer port;
a nebulizing part for nebulizing said liquid supplied from said measuring chamber via a liquid supply port;
a piston for opening and closing said liquid transfer port and pushing said liquid in said measuring chamber, thereby supplying said nebulizing part with said liquid via said liquid supply port;
a piston driving part for driving said piston;
an inhalation port for a user to inhale said liquid nebulized by said nebulizing part; and
inhalation sensing means for sensing said user inhaling from said inhalation port.

25. The liquid nebulizing apparatus according to claim 24, further comprising
nebulizing operation initiating means for initiating a nebulizing operation of said liquid in said nebulizing part in response to said inhalation sensing means sensing an inhalation of said user.

26. The liquid nebulizing apparatus according to claim 24, further comprising
liquid supply means for supplying said nebulizing part with said liquid in response to said inhalation sensing means sensing the inhalation of said user.

27. The liquid nebulizing apparatus according to claim 24, wherein said piston driving part includes a spiral spring as elasticity means for applying elastic force tosaid piston.

## Amended claims

### Amended claims under Art. 19.1 PCT

**1.** (amended) Aliquid nebulizing apparatus, comprising:
a primary reservoir for storing liquid to be nebulized;
a nebulizing part for nebulizing the liquid in said primary reservoir; a secondary reservoir provided between said primary reservoir and said nebulizing part for measuring the liquid from said primary reservoir by an amount to be transferred to said nebulizing part;
a primary liquid transfer part for transferring the liquid in said primary reservoir to said secondary reservoir;
a secondary liquid transfer part for transferring the measured liquid in said secondary reservoir to said nebulizer part; and
an operation part for operating said secondary liquid transfer part; wherein
said nebulizing part is set to initiate a nebulizing operation before said nebulization part is supplied with the liquid in accordance with the operation of said operation part.

**2.** The liquid nebulizing apparatus according to claim 1, wherein said primary liquid transfer part and said secondary liquid transfer part respectively transfer the liquid relative to an operation of said operation part.

**3.** The liquid nebulizing apparatus according to claim 1, wherein said nebulizing part initiates a liquid nebulizing operation simultaneously with an operation of said operation part.

**4.** (cancelled)

**5.** The liquid nebulizing apparatus according to claim 1, wherein said secondary reservoir is set to be supplied with the liquid from said primary reservoir when the liquid transfer operation to said nebulizing part in accordance with the operation of said operation part is completed.

**6.** The liquid nebulizing apparatus according to claim 1, wherein said secondary reservoir is closed except for when transferring liquid to said nebulizing part.

**7.** The liquid nebulizing apparatus according to claim 1, wherein said primary reservoir, said nebulizing part, said secondary reservoir, said primary liquid transfer part and said secondary liquid transfer part are integrally formed as a liquid supply unit, said primary reservoir being removable from said liquid supply unit.

**8.** (amended) A liquid nebulizing apparatus, comprising:
a reservoir chamber for storing liquid;
a measuring chamber to which. a prescribed amount of said liquid is transferred from said reservoir chamber via a liquid transfer port;
a nebulizing part for nebulizing said liquid supplied from said measuring chamber via a liquid supply port;
a piston for opening and closing said liquid transfer port and pushing said liquid in said measuring chamber to supply said nebulizing part with said liquid via said liquid supply port;
a piston drive part for driving said piston at a constant rate; and
a valve determining one end of said measuring chamber and supported by an elastic member, said valve being moved by said piston pushing said liquid, thereby opening said liquid supply port to supply said nebulizing part with said liquid.

**9.** (cancelled)

**10.** The liquid nebulizing apparatus according to claim 8, wherein said nebulizing part includes a mesh member and an oscillator and supplied with said liquid from said measuring chamber.

**11.** The liquid nebulizing apparatus according to claim 8, wherein
said piston driving part includes at least one element selected from the group consisting of elasticity means for applying elastic force to said piston, a motor and a solenoid.

**12.** The liquid nebulizing apparatus according to claim 8, further comprising:
lock means for fixing said piston at a position so as to be able to open said liquid transfer port and transfer said liquid into said measuring chamber from said reservoir chamber; and
a release switch for releasing the fixed state brought by said lock means and for actuating said piston driving part to drive said piston.

**13.** The liquid nebulizing apparatus according to claim 12, further comprising:
lock release sensing means for sensing that the fixed state brought by said lock means is released by said release switch; and
nebulizing operation initiating means for initiating a nebulizing operation of said liquid after said lock release sensing means senses that the fixed state brought by said lock means is released by said release switch.

**14.** , The liquid nebulizing apparatus according to claim 8, wherein said piston driving part includes a first rod, a second rod for pushing said piston, and a connecting member for connecting said first rod and said second rod, and
a moving direction of said first rod and a moving direction of said second rod are set to be different,

**15.** The liquid nebulizing apparatus according to claim 8, further comprising:
a housing having said reservoir chamber inside; and
a cylinder having said measuring chamber inside and receiving said piston; wherein
said cylinder and said piston driving part are removably mounted to said housing

**16.** The liquid nebulizing apparatus according to claim 8, wherein said piston driving part has a spiral spring as elasticity means for applying elastic force to said piston.

**17.** A liquid nebulizing apparatus, comprising:
a reservoir chamber for storing liquid;
